(19) Europäisches Patentamt
European Patent Office
Office européen des brevets

(11) **EP 3 372 152 B1**

(12) **EUROPEAN PATENT SPECIFICATION**

(45) Date of publication and mention
of the grant of the patent:
**17.02.2021 Bulletin 2021/07**

(51) Int Cl.:
*A61B 5/02* *(2006.01)*        *A61B 5/00* *(2006.01)*
*A61B 5/024* *(2006.01)*

(21) Application number: **15907815.3**

(86) International application number:
**PCT/JP2015/081239**

(22) Date of filing: **05.11.2015**

(87) International publication number:
**WO 2017/077629 (11.05.2017 Gazette 2017/19)**

(54) **APPARATUS, METHOD AND COMPUTER PROGRAM FOR DETERMINING WHETHER A MEASURED PULSE WAVEFORM CONTAINS NOISE**

VORRICHTUNG, VERFAHREN UND COMPUTERPROGRAMM ZUR BESTIMMUNG, OB EINE GEMESSENE PULSWELLENFORM RAUSCH ENTHÄLT

DISPOSITIF, PROCÉDÉ ET PROGRAMME INFORMATIQUE PERMETTANT DE DÉTERMINER SI UNE FORME D'ONDE IMPULSIONNELLE MESURÉE CONTIENT DU BRUIT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.09.2018 Bulletin 2018/37**

(73) Proprietor: **Fujitsu Limited**
**Kawasaki-shi, Kanagawa 211-8588 (JP)**

(72) Inventors:
• **UCHIDA, Daisuke**
**Kawasaki-shi**
**Kanagawa 211-8588 (JP)**
• **MORI, Tatsuya**
**Kawasaki-shi**
**Kanagawa 211-8588 (JP)**
• **MAEDA, Kazuho**
**Kawasaki-shi**
**Kanagawa 211-8588 (JP)**
• **INOMATA, Akihiro**
**Kawasaki-shi**
**Kanagawa 211-8588 (JP)**

(74) Representative: **Hoffmann Eitle**
**Patent- und Rechtsanwälte PartmbB**
**Arabellastraße 30**
**81925 München (DE)**

(56) References cited:
**JP-A- 2007 117 591      JP-A- 2007 125 366**
**JP-A- 2010 075 461**

EP 3 372 152 B1

**Description**

FIELD

The embodiments discussed herein are related to a determination apparatus, a determination method, and a determination program for determining whether a pulse waveform contains noise.

BACKGROUND

There are conventional techniques of measuring pulse waves. The pulse wave represents a volumetric change caused by inflow of blood into a certain portion of a body tissue. The pulse wave is one kind of biological information, and is an important indicator for checking a human's vital sign. Examples of the method of measuring the pulse wave include a photoelectric pulse wave measurement method and a facial pulse measurement method.

According one conventional art, measurement values of a patient's blood variable provided by a sensor are stored as a curve for a time t, an average value is derived from the curve, and the average value is used to find a physiological parameter. According to another conventional art, a false peak per pulse is detected, and a pulse wave interval corresponding to a heartbeat is measured from which the influence of the false peak is removed.

Moreover, according still another technique, for example, a change in the detected pulse waveform is monitored, and it is determined that irregular pulse occurs when the continuity of the change in the pulse waveform breaks. According to further another technique, pulse waves of a subject are detected, and a change in the interval between two consecutive pulse waves, and a product of the pulse wave amplitude and the pulse wave interval, or a ratio between the pulse wave amplitude and the pulse wave interval are compared with respective thresholds to determine a singular value of the pulse wave interval

Relevant prior art is disclosed in documents JP 2009-183715, JP 2012-161556, WO 97/38626, JP 2010-75461 and JP 2007-125366.

SUMMARY

TECHNICAL PROBLEM

However, according to the conventional techniques, it is difficult to determine whether or not the measured pulse waveform contains noise caused by body motion (body movement) and so on.

The object of the present disclosure is to provide a determination apparatus, a determination method, and a determination program that enables a pulse waveform containing noise to be identified.

SOLUTION TO PROBLEM

The above objective is achieved by the apparatuses, the methods and the computer programs as defined in the appended claims 1 to 15.

ADVANTAGEOUS EFFECTS OF INVENTION

One aspect of the present disclosure has the advantageous effect of enabling a pulse waveform containing noise to be identified.

BRIEF DESCRIPTION OF DRAWINGS

FIG. 1 illustrates an example of a determination method in accordance with Embodiment 1.
FIG. 2 illustrates a specific example of a pulse waveform.
FIG. 3 illustrates an example of configuration of a pulse wave analysis system 300.
FIG. 4 is a block diagram illustrating an example of hardware configuration of a pulse wave analysis apparatus 301.
FIG. 5 is a block diagram illustrating hardware configuration of a server 302.
FIG. 6 illustrates an example of contents stored in a pulse waveform DB 320.
FIG. 7 illustrates an example of contents stored in an analysis result DB 330.
FIG. 8 is a block diagram illustrating an example of functional configuration of the pulse wave analysis apparatus 301 in accordance with Embodiment 1.

FIG. 9 illustrates a specific example of an outlier.
FIG. 10 illustrates a specific example (1) of a pulse wave analysis result.
FIG. 11 illustrates a specific example (2) of the pulse wave analysis result.
FIG. 12 illustrates a specific example of a pulse wave area.
FIG. 13 illustrates an example of contents stored in an individual pulse waveform table 1300.
FIG. 14 illustrates an example of contents stored in an individual analysis result table 1400.
FIG. 15 illustrates an example of decision of a threshold Th and a predetermined range R2.
FIG. 16 illustrates an example of contents stored in a threshold table 1600.
FIG. 17A is a flow chart (1) illustrating an example of a pulse wave analysis processing procedure of the pulse wave analysis apparatus 301 in accordance with Embodiment 1.
FIG. 17B is a flow chart (2) illustrating an example of a pulse wave analysis processing procedure of the pulse wave analysis apparatus 301 in accordance with Embodiment 1.
FIG. 18 is a block diagram illustrating an example of functional configuration of the pulse wave analysis apparatus 301 in accordance with Embodiment 2.
FIG. 19 illustrates a temporal change in the pulse waveform.
FIG. 20 illustrates a relationship between an amplitude ratio between adjacent pulse waves and a pulse wave interval in each section Sk.
FIG. 21 illustrates a specific example of an approximate plane.
FIG. 22A is a flow chart (1) illustrating an example of a pulse wave analysis processing procedure of the pulse wave analysis apparatus 301 in accordance with Embodiment 2.
FIG. 22B is a flow chart (2) illustrating an example of a pulse wave analysis processing procedure of the pulse wave analysis apparatus 301 in accordance with Embodiment 2.

DESCRIPTION OF EMBODIMENTS

Referring to figures, embodiments of a determination apparatus, a determination method, and a determination program according to the present disclosure will be described below.

(Embodiment 1)

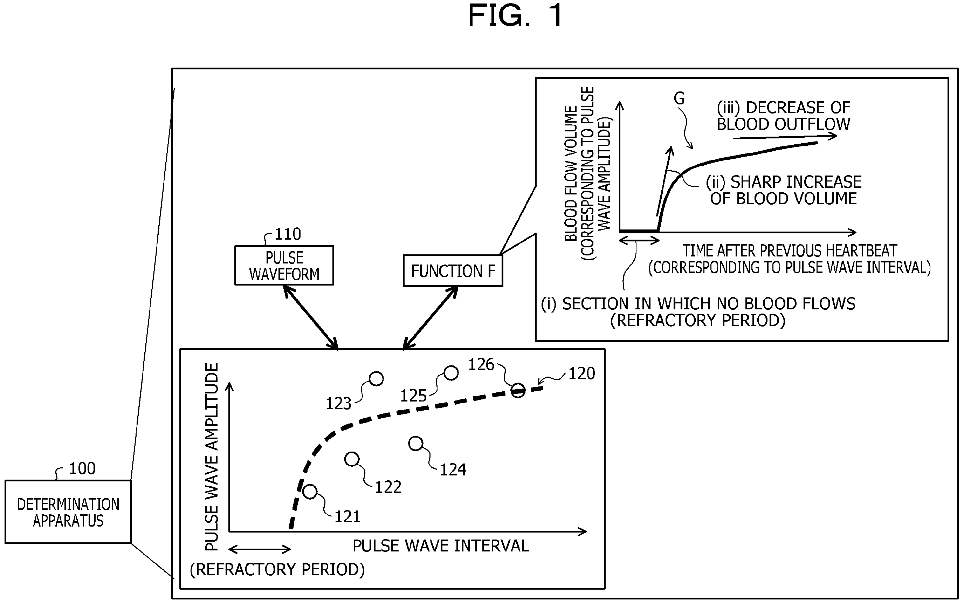

FIG. 1 illustrates an embodiment of a determination method in accordance with Embodiment 1. In FIG. 1, a determination apparatus 100 is a computer that assists analysis measured subject's pulse waves. The subject is a person whose pulse wave is measured. The pulse wave represents a volumetric change caused by inflow of blood into a certain portion of a body tissue, and is taken from a body surface in the form of waveform.
The pulse wave is one of important indicators for determining human's vital signs. For example, a pulse rate is an indicator that indicates the subject's state of health. When an excessively high pulse rate (so-called tachycardia) frequently occurs, or continues without any psychological stress, abnormal cardiac function is suspected. The irregular pulse with irregular pulse rhythm may lead to serious diseases such as heart failure and cerebral infarction.
As described above, a lot of information on the person's state of health may be acquired based on pulse waves. For this reason, it is desirable that pulse waves may be measured ordinarily, continually, and subconsciously by the most convenient measurement method, and the measurement method and measurement equipment are noninvasive. Here, examples of the method of measuring pulse waves include a photoelectric pulse wave measurement method and a face pulse measurement.
According to the photoelectric pulse wave measurement method, pulse waves are measured by irradiating peripheral blood vessels of a finger, an ear lobe, or the like with an infrared radiation, and catching a cyclic optical change in reflected light, which occurs due to blood flow and light absorption characteristics. According to the face pulse measurement method, pulse waves are measured by making use of the property that hemoglobin in blood absorbs green light to catch a change in the brightness on the face surface, the change being is caused by blood flow.
However, the pulse waveform measured by the photoelectric pulse wave measurement method or the face pulse measurement may include noise generated from body motion (body movement). For example, according to the face pulse measurement, a signal of pulse wave is feeble and thus, noise easily comes in. In performing pulse analysis such as irregular pulse determination or pulse rate determination, when noise may not be removed from the measured pulse waveform, the analysis accuracy of the pulse wave may not be ensured. However, the pulse waveform containing noise is an irregular form like the irregular pulse and thus, is difficult to distinguish from the irregular pulse according to the conventional techniques.
Thus, Embodiment 1 describes a determination method enabling a pulse waveform containing noise caused by a body motion or the like to be identified in the measured pulse waveform of the subject. An example of processing of the determination apparatus 100 will be described below.

(1) The determination apparatus 100 acquires the pulse waveform indicating a temporal change in the measured subject's pulse waves. The pulse waves may be measured by any suitable measurement method such as the photoelectric pulse wave measurement method and the face pulse measurement. Specifically, for example, the determination apparatus 100 acquires a pulse waveform 110 indicating a temporal change in the subject's pulse waves measured for a predetermined time (for example, 10 seconds).

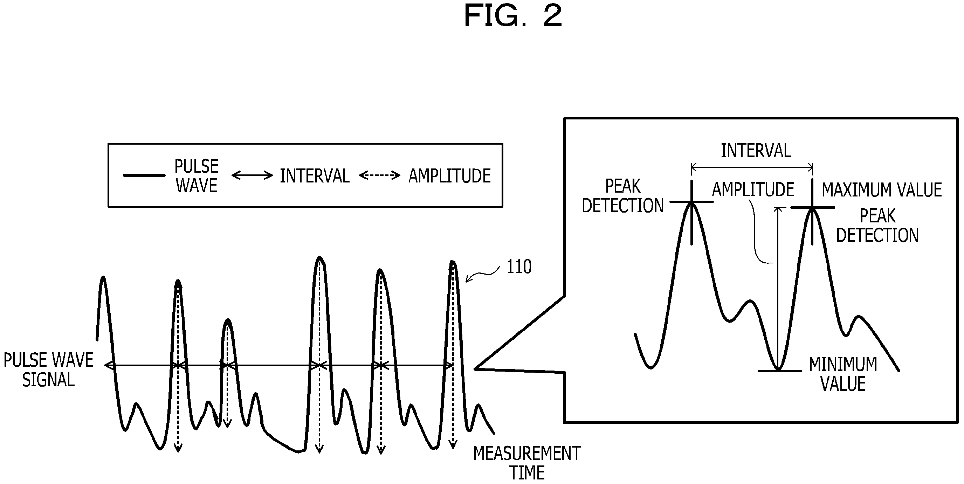

Here, referring to FIG. 2, a specific example of a pulse waveform 110 is described. The pulse waveform may be acquired at measurement of pulse waves in real time, or may indicate a temporal change in the measured pulse waves.

FIG. 2 illustrates a specific example of the pulse waveform. In FIG. 2, the pulse waveform 110 indicates the temporal change in the measured pulse waves. In FIG. 2, solid double-headed arrows each represent an interval between the pulse waves. Dotted double-headed arrows each represent the pulse wave amplitude. The interval between the pulse waves (pulse wave interval) is, for example, an interval between consecutive peaks in time series. The pulse wave amplitude is, for example, a difference between a maximum value (peak) and a minimum value of the pulse wave signal.

(2) The determination apparatus 100 calculates a value indicating a matching degree between the acquired pulse waveform and a model expressed by a function F. The function F indicates the relationship between an interval between heartbeats and a blood flow volume, for example, a function indicating a temporal change in the blood flow volume with heartbeat (contraction and relaxation). In the following description, the matching degree between the pulse waveform and the model expressed by the function F may be also referred to as "matching score".

Here, for example, the temporal change in the blood flow volume with heartbeat is represented as a graph G, and has the following features (i) to (iii). A horizontal axis indicates the temporal change from the previous heartbeat to the next heartbeat, and corresponds to the interval between pulse waves. A vertical axis indicates the blood flow volume with a heartbeat, and the blood flow volume is proportional to the pulse wave amplitude.

(i) For a certain period after a heartbeat, a section in which no blood flows out with the next heartbeat suspended. The section is referred to as "refractory period", and is about 150 [ms] to 300 [ms]. (ii) Immediately after contraction of the heart, the pressure difference between the inside of the heart and a vein is so large that the blood abruptly flows out. (iii) As the heart relaxes, the pressure difference between the inside of the heart and the vein becomes smaller, decreasing the outflow of blood.

The function F is a model of the above features (i) to (iii), and indicates the temporal change in the blood flow volume that monotonously increases a certain period (corresponding to the above-mentioned "refractory period") after the previous heartbeat. In other words, the function F is a function representing the correspondence between the pulse wave amplitude (or pulse wave area) and the pulse interval.

Specifically, for example, the determination apparatus 100 identifies multiple pairs of the pulse wave amplitude and the pulse wave interval, based on the acquired pulse waveform. Then, the determination apparatus 100 performs regression analysis based on the multiple pairs of the pulse wave amplitude and the pulse wave interval, thereby calculating a determination coefficient of an approximate curve 120 (function F).

The determination coefficient is used as a measure of a good fit to a regression equation found from sampled values. For this reason, the determination coefficient is calculated as the matching score between the pulse waveform and the model expressed as the function F. In the example illustrated in FIG. 1, it is assumed that regression analysis is performed based on the multiple pairs 121 to 126 of the pulse wave amplitude and the pulse wave intervals, resulting in that "0.45" is calculated as the determination coefficient of the approximate curve 120.

(3) The determination apparatus 100 determines whether or not the pulse waveform contains noise, based on the calculated matching score. Here, as the matching score between the pulse waveform and the model expressed as the function F is higher, the acquired pulse waveform reflects the pulse waves of the subject rather than noise caused by body motion and so on.

For this reason, for example, the determination apparatus 100 may determine that the pulse waveform contains noise, when the matching score between the calculated pulse waveform and the model expressed as the function F is less than a threshold. The threshold (corresponding to below-mentioned "threshold Th") may be set to any value, for example, about 0.5.

Here, the threshold is set to "0.5". In this case, in the example illustrated in FIG. 1, the determination coefficient of "0.45" indicating the matching score between the calculated pulse waveform 110 and the model expressed as the function F is less than the threshold, the determination apparatus 100 determines that the pulse waveform 110 contains noise.

(4) The determination apparatus 100 outputs a determination result. Specifically, for example, the determination apparatus 100 may associate the acquired pulse waveform 110 with the determination result that the pulse waveform 110 contains noise, and output the result.

As described above, the determination apparatus 100 may determine whether or not the pulse waveform contains noise by modelling the temporal change in the blood flow volume with heartbeat as the function F, and determining whether or not the measured pulse waveform (for example, the pulse waveform 110) matches with the model. This enables the pulse waveform containing noise to be identified, and thereby improves the analysis accuracy of pulse waves such as the irregular pulse determination and the pulse rate determination.

In the above description, the determination apparatus 100 determines whether or not the pulse waveform contains noise, based on the calculated matching score and however, the present disclosure is not limited to this. For example, the determination apparatus 100 may associate the calculated matching score with the acquired pulse waveform, and output the matching score. Therefore, the user may determine whether or not the pulse waveform contains noise, based on the outputted matching score associated with the pulse waveform.

(Configuration of pulse wave analysis system 300)

Next, an example of configuration of a pulse wave analysis system 300 in accordance with Embodiment 1 will be described. Here, the determination apparatus 100 illustrated in FIG. 1 is applied to a pulse wave analysis apparatus 301 of the pulse wave analysis system 300.

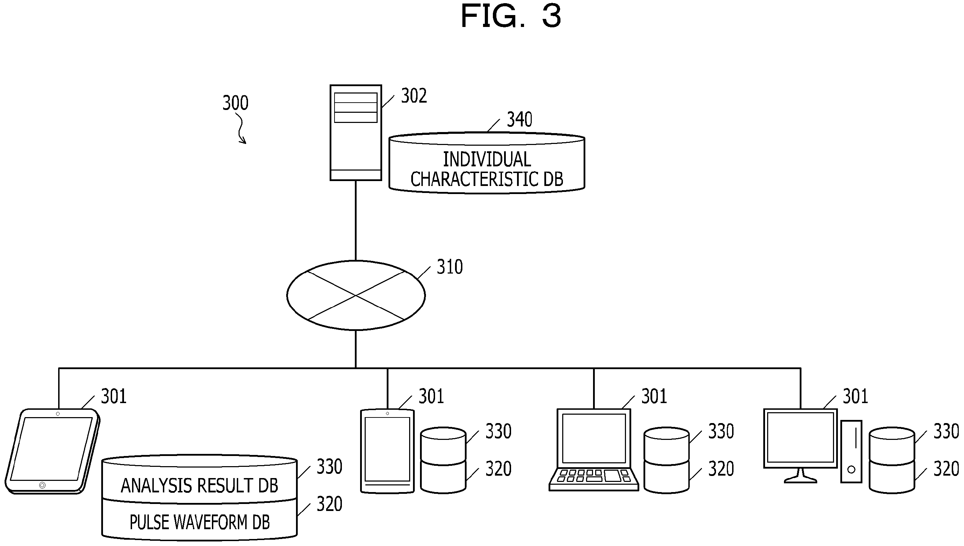

FIG. 3 illustrates the example of the configuration of the pulse wave analysis system 300. In FIG. 3, the pulse wave analysis system 300 includes multiple (four In FIG. 3) pulse wave analysis apparatuses 301, and a server 302. In the pulse wave analysis system 300, the pulse wave analysis apparatuses 301 are connected to the server 302 via a wired or wireless network 310. The network 310 is local area network (LAN), wide area network (WAN), the Internet, or the like.

The pulse wave analysis apparatuses 301 each are a computer that has a pulse waveform database (DB) 320 and an analysis result DB 330, and serves to analyze the pulse wave. Examples of pulse wave analysis apparatus 301 include a tablet terminal, a smart phone, a personal digital assistant (PDA), a wearable terminal, a notebook personal computer (PC), and a desktop PC.

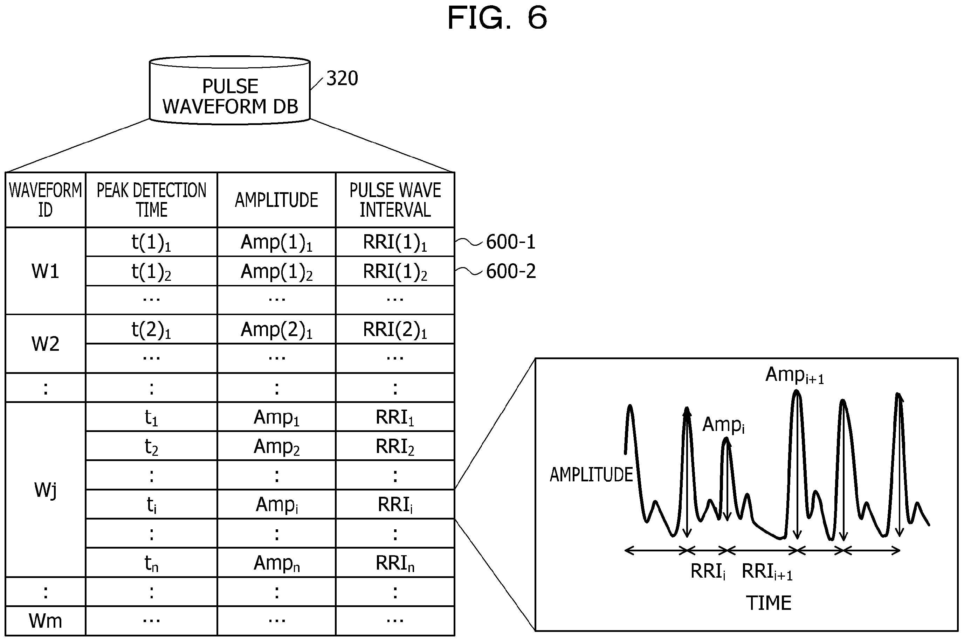

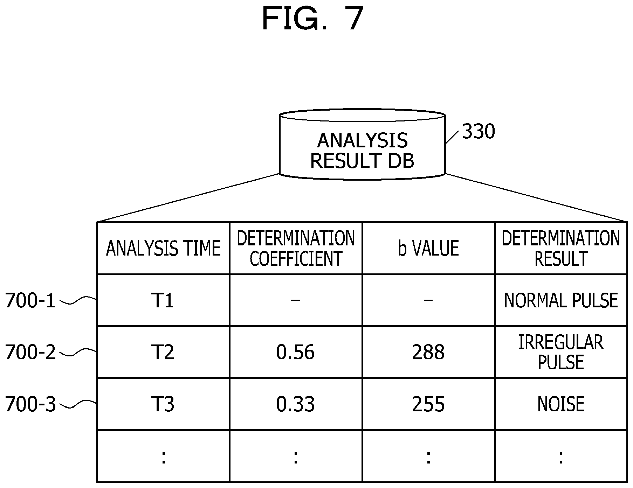

The pulse waveform DB 320 stores the pulse wave amplitude and the pulse wave interval, which are measured from the subject. The analysis result DB 330 stores results of the pulse wave analysis. Contents stored in the pulse waveform DB 320 and the analysis result DB 330 will be described below with reference to FIGs. 6 and 7.

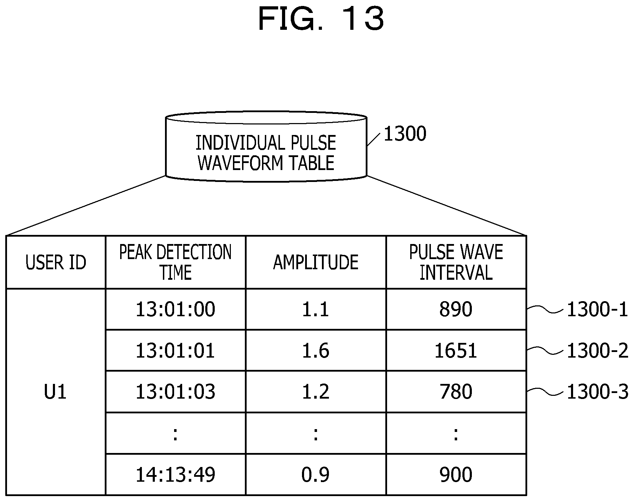

The server 302 is a computer that has an individual characteristic DB 340, and manages analysis results of the pulse waveform and the pulse wave for each subject. The individual characteristic DB 340 includes an individual pulse waveform table 1300, an individual analysis result table 1400, and a threshold table 1600 in FIGs. 13 to 16.

Here, the pulse wave analysis system 300 includes the pulse wave analysis apparatus 301 and the server 302 and however, the present disclosure is not limited to this. For example, each of the pulse wave analysis apparatuses 301 may achieve the function of the pulse wave analysis system 300. In this case, the pulse wave analysis apparatuses 301 may have their own individual characteristic DBs 340.

(Hardware configuration of pulse wave analysis apparatus 301)

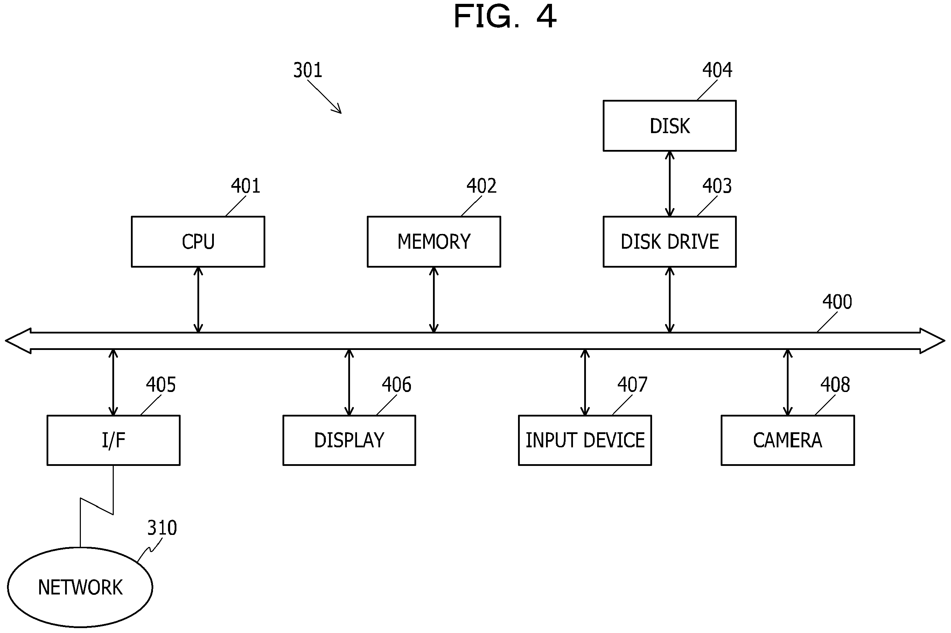

FIG. 4 is a block diagram illustrating an example of hardware configuration of pulse wave analysis apparatus 301. In FIG. 4, the pulse wave analysis apparatuses 301 each have a central processing unit (CPU) 401, a memory 402, a disk drive 403, a disk 404, an interface (I/F) 405, a display 406, an input device 407, and a camera 408. The constituents are connected to each other via a bus 400.

Here, the CPU 401 controls the entire pulse wave analysis apparatus 301. The memory 402 has, for example, a read only memory (ROM), a random access memory (RAM), and a flash ROM. Specifically, for example, the flash ROM and the ROM store various programs, and the RAM is used as a work area for the CPU 401. The programs stored in the memory 402 are loaded into the CPU 401, causing the CPU 401 to executed coded processing.

The disk drive 403 controls reading/writing of data from/to the disk 404 under control of the CPU 401. The disk 404 stores data written under control of the disk drive 403. Examples of the disk 404 include a magnetic disk and an optical disk.

The I/F 405 is connected to the network 310 via a communication line, and is connected to another device (for example, the server 302 illustrated in FIG. 3) via the network 310. The I/F 405 serves as an interface between the network 310 and the inside of the apparatus of its own, and controls data input/output with respect to another device.

The display 406 displays a cursor, an icons or tool box, and data including documents, images, and functional information. Examples of the display 406 include a liquid crystal display, an organic electroluminescence (EL) display, and a cathode ray tube (CRT).

The input device 407 has keys for inputting characters, numerals, and various instructions to input data. The input device 407 may be a keyboard, a mouse, or touch panel-type input pad or numerical keypad.

The camera 408 is an imaging device that takes static or moving images, and outputs the taken images as image data. The images taken by use of the camera 408 are recorded as image data in a storage device such as the memory 402 or the disk 404. For example, a digital camera or a Web camera as the camera 408 may be connected via an external terminal of the pulse wave analysis apparatus 301.

The pulse wave analysis apparatus 301 may have, in addition to the above-mentioned constituents, a photoelectric pulse wave sensor that measures pulse waves by photoelectric pulse wave measurement method. The pulse wave analysis apparatus 301 may omit the disk drive 403, the disk 404, and the camera 408 in the above-mentioned constituents.

(Example of hardware configuration of server 302)

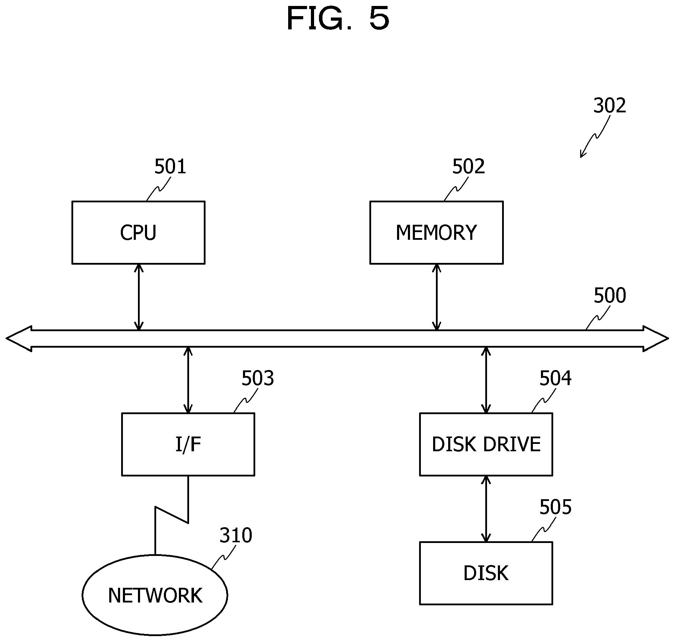

FIG. 5 is a block diagram illustrating an example of hardware configuration of server 302. In FIG. 5, the server 302 has a CPU 501, a memory 502, an I/F 503, a disk drive 504, and a disk 505. The constituents are connected to each other via a bus 500.

Here, the CPU 501 controls the entire server 302. The memory 502 has, for example, a ROM, a RAM, and a flash ROM. Specifically, for example, the flash ROM and the ROM store various programs, and the RAM is used as a work area for the CPU 501. The programs stored in the memory 502 are loaded into the CPU 501, causing the CPU 501 to executed coded processing.

The I/F 503 is connected to the network 310 via a communication line, and is connected to another device (for example, the pulse wave analysis apparatus 301 illustrated in FIG. 2) via the network 310. The I/F 503 serves as an interface between the network 310 and the inside of the apparatus of its own, and controls data input/output with respect to another device. Examples of the I/F 503 include a modem and a LAN adaptor.

The disk drive 504 controls reading/writing of data from/to the disk 505 under control of the CPU 501. The disk 505 stores data written under control of the disk drive 504. Examples of the disk 505 include a magnetic disk and an optical disk.

The server 302 may have, in addition to the above-mentioned constituents, a solid state drive (SSD), a keyboard, a mouse, and a display.

(Contents stored in pulse waveform DB 320)

Here, the contents stored in the pulse waveform DB 320 of the pulse wave analysis apparatus 301 will be described. The pulse waveform DB 320 is stored in a storage device such as the memory 402 and the disk 404 in FIG. 4.

FIG. 6 illustrates an example of the contents stored in the pulse waveform DB 320. In FIG. 6, the pulse waveform DB 320 has fields for waveform ID, peak detection time, amplitude, and pulse wave interval, and sets information in each field to store pulse wave information (for example, pulse wave information 600-1, 600-2) as records.

Here, the waveform ID is an identifier that uniquely identifies the pulse waveform. The peak detection time is a time when a peak of the pulse wave is detected. For example, the peak detection time is represented as a time of day when the peak is detected. The amplitude is a difference between a maximum value (peak) and a minimum value of the pulse wave signal. The pulse wave interval is an interval between adjacent peaks (two consecutive peaks in time series).

In the following description, any pulse waveform among pulse waveforms W1 to Wm may be referred to as "pulse waveform Wj" (j = 1, 2, ..., m). In the pulse waveform Wj, the peak detection time included may be referred to as "peak detection times $t_1$ to $t_n$", the amplitude of the pulse wave signal may be referred to as "amplitudes $Amp_i$ to $Amp_n$", and the pulse wave interval may be referred to as "pulse wave intervals $RRI_1$ to $RRI_n$" (n is a natural number).

(Contents stored in analysis result DB 330)

Next, contents stored in the analysis result DB 330 of the pulse wave analysis apparatus 301 will be described. The analysis result DB 330 is stored in a storage device such as the memory 402 and the disk 404 in FIG. 4.

FIG. 7 illustrates an example of the contents stored in the analysis result DB 330. In FIG. 7, the analysis result DB 330 has fields for analysis time, determination coefficient, b value, and determination result, and sets information in each field, thereby storing analysis results (for example, analysis result 700-1 to 700-3) as records.

Here, the analysis time is a time when the pulse wave indicated by the pulse waveform to be analyzed is measured. For example, the analysis time is represented by a time of day when the pulse wave is measured. The determination coefficient is a value indicating a matching degree between the pulse waveform to be analyzed and the model expressed as the function F, which represents a temporal change in the blood flow volume with heartbeat (contraction and relaxation). The b value is a coefficient value b included in a below-mentioned equation (1). The determination result indicates what the pulse waveform to be analyzed is "normal pulse", "irregular pulse", or "noise".

(Functional configuration of pulse wave analysis apparatus 301)

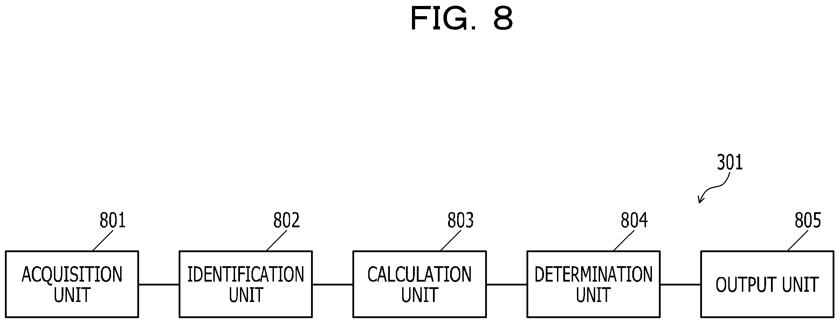

FIG. 8 is a block diagram illustrating an example of functional configuration of the pulse wave analysis apparatus 301 in accordance with Embodiment 1. In FIG. 8, the pulse wave analysis apparatus 301 includes an acquisition unit 801, an identification unit 802, a calculation unit 803, a determination unit 804, and an output unit 805. The acquisition unit 801 to output unit 805 function as control units, and specifically, cause the CPU 401 to execute the programs stored in the storage devices such as the memory 402 and the disk 404 in FIG. 4, or use the I/F 405 to achieve their functions. Processing results of the function units are stored in the storage devices such as the memory 402 and the disk 404.

The acquisition unit 801 acquires the pulse waveform representing the temporal change in the pulse wave measured from the subject. Specifically, for example, the acquisition unit 801 may acquire the pulse waveform $Wj$ indicating the temporal change in pulse waves measured in in each of sections divided along a time axis. A time width of each section may be set to any value, for example, 10 seconds.

Here, the pulse wave may be measured by using any existing measurement method. For example, the pulse wave analysis apparatus 301 may measure a brightness change signal included in an image of a body section (for example, face), which is taken by use of the camera 408 (See FIG. 4), as the pulse wave of a living body. In this case, the acquisition unit 801 acquires the pulse waveform indicating the temporal change in the pulse waves measured in the apparatus of its own.

The face pulse measurement is well-known technique and thus detailed description thereof is omitted. However, Japanese Laid-open Patent Publication No. 2014-200390 may be referred to for the technique.

The acquisition unit 801 also may acquire the pulse waveform indicating a temporal change in pulse waves measured by a photoelectric pulse wave sensor not illustrated. Further, the acquisition unit 801 may acquire the pulse waveform indicating a temporal change in subject's measured pulse waves from an external computer via the network 310.

The identification unit 802 identifies the pulse wave interval of the measured pulse waves, based on the acquired pulse waveform. Specifically, for example, first, the identification unit 802 detects the peak of the acquired pulse waveform $Wj$, and identifies the peak detection time $t_i$ of each detected peak. Peak detection is the processing of detecting a peak (maximum value) at which the pulse wave signal is equal or greater than a specified value.

Then, identification unit 802 identifies a peak detection time interval $(t_i - t_{i-1})$ between consecutive peaks in time series, as the pulse wave interval $RRI_i$. The identified pulse wave interval $RRI_i$ is associated with, for example, the waveform ID and the peak detection time $t_i$ of the pulse waveform $Wj$, and is stored in the pulse waveform DB 320 illustrated in FIG. 6.

The identification unit 802 identifies the measured pulse wave amplitude, based on the acquired pulse waveform. Specifically, for example, the identification unit 802 detects a maximum value (peak) and a minimum value of the pulse waveform $Wj$, and identifies a difference between the consecutive maximum value and minimum value in time series (or the consecutive minimum value and maximum value in time series) as the amplitude $Amp_i$. For example, the identified amplitude $Amp_i$ is associated with the waveform ID and the peak detection time $t_i$ of the pulse waveform $Wj$, and is stored in the pulse waveform DB 320 illustrated in FIG. 6.

The calculation unit 803 calculates a value indicating the variation degree between the identified pulse wave intervals. The variation degree between pulse wave intervals indicates how much the interval between adjacent pulse waves in the pulse waveform varies. In the following description, the value indicating the variation degree between pulse wave intervals is also referred to as "the pulse wave interval variation score".

Specifically, for example, the calculation unit 803 may refer to the pulse waveform DB 320 to calculate a pulse wave interval ratio $(RRI_{i+1}/RRI_i)$ of adjacent pulse waves in the pulse waveform $Wj$, as the pulse wave interval variation score. In this calculation, the calculation unit 803 may calculate all the pulse wave interval ratios between the pulse waves included in the pulse waveform $Wj$.

The determination unit 804 determines whether or not the acquired pulse waveform indicates the normal pulse, based on the calculated pulse wave interval variation score. Here, the pulse wave interval generally matches with the interval between heart beats (heartbeat interval). Typically, the pulse wave interval generally varies. However, a healthy person has no rapid change in pulse waves, and experiences heartbeats of substantially fixed rhythm.

For this reason, the determination unit 804 may determine that the acquired pulse waveform $Wj$ indicates the normal pulse, for example, when the pulse wave interval variation score falls within a predetermined range R1. On the other hand, the determination unit 804 may determine that the acquired pulse waveform $Wj$ does not indicate the normal pulse, when the pulse wave interval variation score falls outside the predetermined range R1.

More specifically, for example, the determination unit 804 may determine that the pulse waveform $Wj$ indicates the normal pulse, when all the pulse wave interval ratios between pulse waves in the pulse waveform $Wj$ fall within the predetermined range R1. For example, the determination unit 804 may determine that the pulse waveform $Wj$ indicates the normal pulse, when the pulse wave interval ratios that occupy a predetermined proportion (for example, 90% or more) in all the pulse wave interval ratios of the pulse waves in the pulse waveform $Wj$ fall within the predetermined range R1.

Thereby, the pulse waveform Wj having a small pulse wave interval variation score may be classified as "normal pulse". The predetermined range R1 may be set to any range. For example, when the pulse wave interval ratio ($RRI_{i+1}/RRI_i$) is set to the pulse wave interval variation score, the predetermined range R1 is set to the range of "$0.9 \leq R1 \leq 1.1$".

For example, the obtained determination result is associated with the analysis time Tj of the pulse waveform Wj, and is stored in the analysis result DB 330 illustrated in FIG. 7.

In the example illustrated in FIG. 7, when it is determined that the pulse waveform W1 indicates the normal pulse, the analysis time T1 and the determination result "normal pulse" of the pulse waveform W1 are set to the field for the analysis time and the field for the determination result in the analysis result DB 330, respectively, and are stored as the analysis result 700-1. However, "-(null)" is set to the fields for the determination coefficient and the b value.

The calculation unit 803 calculates the matching score between the acquired pulse waveform and the model expressed as the function F. Here, as described above, the function F is a function that indicates the temporal change in the blood flow volume with heartbeat (contraction and relaxation). For example, the following equation (1) may be used as the function F.

Wherein, Amp denotes the pulse wave amplitude. RRI denotes the pulse wave interval. a and b are coefficients. That is, the following equation (1) is a function which increases monotonously in a convex upward shape while passing the origin, and which represents the relationship between the pulse wave amplitude Amp and a value of the pulse wave interval RRI minus the coefficient b (b value).

[Equation 1]

$$Amp = \sqrt{a * (RRI - b)} \quad ... (1)$$

Specifically, for example, first, when it is determined that the pulse waveform Wj does not indicate the normal pulse, the calculation unit 803 refers to the pulse waveform DB 320 to identify multiple pairs of the pulse wave amplitude $Amp_i$ and the pulse wave interval $RRI_i$ in the pulse waveform Wj. Describing in more detail, for example, the calculation unit 803 identifies each of the pairs of the amplitudes $Amp_1$ to $Amp_n$ and the pulse wave intervals $RRI_1$ to $RRI_n$ in the pulse waveform Wj.

In the following description, the pairs of the amplitudes $Amp_1$ to $Amp_n$ and the pulse wave intervals $RRI_1$ to $RRI_n$ may be referred to as "pairs P1 to Pn".

Using the equation (1), the calculation unit 803 performs regression analysis based on the identified pairs P1 to Pn to calculate values of the coefficients a, b and the determination coefficient. Here, the determination coefficient is a value indicating how an independent variable (explanatory variable) contributes a dependent variable (explained variable), which is also referred to as "contribution ratio".

The determination coefficient is used as a measure of a good fit to the regression equation found from a sample value. For this reason, the determination coefficient calculates the matching score of the pulse waveform Wj. The determination coefficient may be found, for example, by using a following equation (2). Wherein, the residual is a difference between the sample value (the amplitude $Amp_i$ with respect to the pulse wave interval $RRI_i$) and a value estimated by the regression equation.

Determination coefficient = 1 - (square sum of residual)/(square

sum of difference from average of sample values) ... (2)

The determination unit 804 determines whether or not the pulse waveform Wj contains noise, based on the calculated matching score. Specifically, for example, the determination unit 804 may determine that the pulse waveform Wj contains noise, when the calculated matching score is less than the threshold Th. The threshold Th may be set to any value, and is set to, for example, about 0.5.

Thereby, the pulse waveform Wj having a low matching degree with the model representing the temporal change in the blood flow volume with heartbeat may be classified as "noise".

Alternatively, for example, the determination unit 804 may determine that the pulse waveform Wj includes the irregular pulse, when it is determined that the pulse waveform Wj does not indicate the normal pulse, and the calculated matching score is less than the threshold Th. Thereby, the pulse waveform Wj having a large pulse wave interval variation score, and a high matching degree with the model representing the temporal change in the blood flow volume with heartbeat may be classified as "irregular pulse".

The determination unit 804 may determine that the pulse waveform Wj contains noise, when the calculated the coefficient value b (b value) falls outside a predetermined range R2. The predetermined range R2 may be set to any range, and is set to, for example, a range of "150 [ms] $\leq$ R2 $\leq$ 300 [ms]" corresponding to the refractory period of the heart.

Thereby, the pulse waveform Wj having the b value corresponding to the refractory period of the heart outside the predetermined range R2 representing the general refractory period may be classified as "irregular pulse".

As described above, for example, the obtained determination result is associated with the analysis time Tj of the pulse waveform Wj, and is stored in the analysis result DB 330.

For example, when it is determined that the pulse waveform W2 includes the irregular pulse, the analysis time T2 and the determination result "irregular pulse" of the pulse waveform W2 are stored in the analysis result DB 330. In the example illustrated in FIG. 7, the analysis time T2 and the determination result "irregular pulse" are set to the field for the analysis time and the field for the determination result in the analysis result DB 330, respectively, and are stored as the analysis result 700-2. The calculated determination coefficient "0.56" and the b value "288" are set to the field for the determination coefficient and the field for the b value in the analysis result 700-2, respectively.

For example, when it is determined that the pulse waveform W3 contains noise, the analysis time T3 and the determination result "noise" of the pulse waveform W3 are stored in the analysis result DB 330. In the example illustrated in FIG. 7, the analysis time T3 and the determination result "noise" are set to the field for the analysis time and the field for the determination result in the analysis result DB 330, respectively, and are stored as the analysis result 700-3. The calculated determination coefficient "0.33" and the b value "255" are set to the field for the determination coefficient and the field for the b value in the analysis result 700-3.

The calculation unit 803 may delete the pair Pi that is an outlier among the pairs P1 to Pn, when the matching score of the calculated pulse waveform Wj is less than the threshold Th, or when the calculated b value falls outside the predetermined range R2. Here, the outlier refers to a value that greatly deviates from a general tendency.

Specifically, for example, referring to the pulse waveform DB 320, the calculation unit 803 may compare the approximate curve (regression equation) found by regression analysis for each of the pairs P1 to Pn, and delete the pair that deviates the most as the outlier. For example, the calculation unit 803 may delete data that deviates from an average of sample values by three times of a standard deviation or more, as the outlier.

Then, calculation unit 803 may delete the pair Pi as the outlier from the pairs P1 to Pn and then, recalculate the pulse wave interval variation score in the deleted pair. This may recalculate the variation score based on data with the outlier removed, improving the accuracy of pulse wave analysis.

Using the above equation (1), the calculation unit 803 may delete the pair Pi as the outlier from the pairs P1 to Pn and then, perform regression analysis based on the pairs with the pair Pi deleted to recalculate values of the coefficients a, b and the determination coefficient. This may recalculate the values of the coefficients a, b and the determination coefficient, based on the data with the outlier removed, improving the accuracy of pulse wave analysis.

However, to perform regression analysis, at least three pieces of data are requested. For this reason, when the number of pairs before deletion of the pair as the outlier is four or more, the values of the coefficients a, b and the determination coefficient may be recalculated.

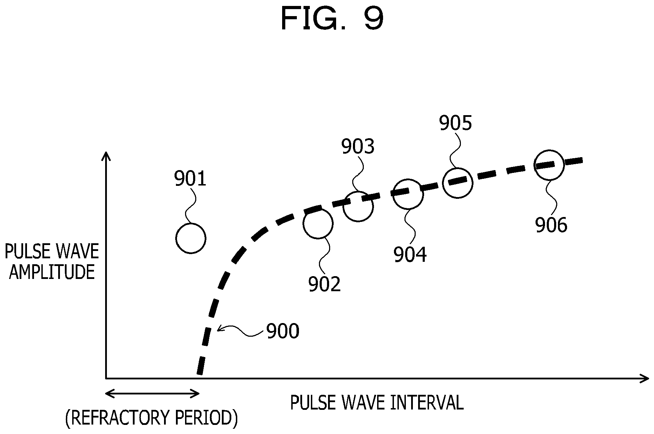

Here, referring to FIG. 9, a specific example of the outlier will be described.

FIG. 9 illustrates a specific example of the outlier. In FIG. 9, a dotted line graph 900 represents an approximate curve found on a certain pulse waveform by regression analysis. The data pieces 901 to 906 correspond to respective pairs of the amplitude Amp and the pulse wave interval RRI in a certain pulse waveform.

In this case, for example, the calculation unit 803 compare each of the data pieces 901 to 906 with the dotted line graph 900, and identifies data that deviates from the dotted line graph 900 the largest as the outlier. In the example illustrated in FIG. 9, the data piece 901 is identified as the outlier. Then, for example, the calculation unit 803 deletes the pulse wave information on the pair of the amplitude Amp and the pulse wave interval RRI, which corresponds to the data piece 901 of the pulse waveform, from the pulse waveform DB 320.

Returning to description with reference to FIG. 8, the output unit 805 outputs the obtained determination result. Specifically, for example, the output unit 805 may refer to the analysis result DB 330 to output a pulse wave analysis result that associates the analysis time Tj of the pulse waveform Wj with the determination result of the pulse waveform Wj. As an output mode, the output unit 805 may display the result on the display 406 (See FIG. 4), output the result to a printer not illustrated, or transmit the result to an external device via the I/F 405.

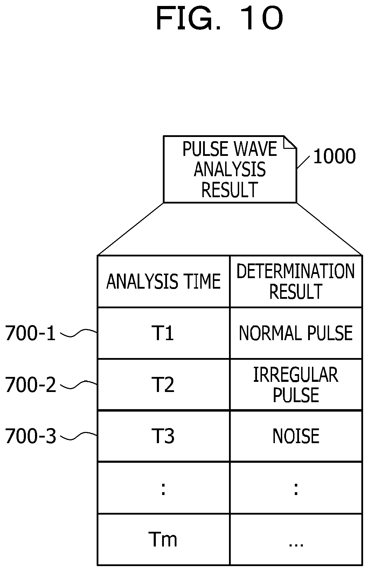

Here, referring to FIG. 10, a specific example of the pulse wave analysis result will be described.

FIG. 10 illustrates a specific example (1) of the pulse wave analysis result. In FIG. 10, in a pulse wave analysis result 1000, analysis times T1 to Tm of the pulse waveforms W1 to Wm are associated with respective determination results of the pulse waveforms W1 to Wm.

The pulse wave analysis result 1000 may determine which of pulse wave "normal pulse", "irregular pulse", and "noise" is detected at each of the analysis times T1 to Tm. The analysis times T1 to Tm each are information identifying a time when each of the pulse waveforms W1 to Wm, that is, a period from the start of measurement to the end of measurement.

For example, the user may recognize that the pulse waveform W1 at the analysis time T1 indicates "normal pulse". The user may recognize that the pulse waveform W2 at the analysis time T2 includes "irregular pulse". The user may recognize that the pulse waveform W3 at the analysis time T3 includes "noise".

The output unit 805 may output the pulse waveform Wj together with the pulse wave analysis result of the pulse waveform Wj. Referring to the pulse waveform DB 320, the output unit 805 may output the peak detection times $t_1$ to $t_n$ of peaks of the pulse waveform Wj as well as the pulse wave analysis result of the pulse waveform Wj.

At this time, the output unit 805 does not output the peak detection time corresponding to the pair deleted as the outlier, among from the peak detection times $t_1$ to $t_n$. For this reason, for example, when it is determined that the pulse waveform Wj contains the irregular pulse, the user may determine that the peak corresponding to remaining undeleted peak detection time is the irregular pulse.

Potential peaks corresponding to the irregular pulse and noise may be narrowed down by appropriately setting the section for acquiring the pulse waveform.

It is assumed that the time width of each section is 10 seconds, and the sections are displaced from each other by 1 second. Given that the determination result of the pulse waveform W1 is "normal pulse" and the determination result of the pulse waveform W2 is "irregular pulse", the user may identify the peak in the remaining section (1 second) that does not overlap the section of the pulse waveform W1 in the section of the pulse waveform W2, as the irregular pulse.

It is assumed that the time width of each section is a length including 10 peaks, and the sections are displaced from each other by one peak. Given that the determination result of the pulse waveform W1 is "normal pulse", and the determination result of the pulse waveform W2 is "irregular pulse", the pulse wave analysis apparatus 301 may identify the last peak other than the peaks in the section of the pulse waveform W1 among the 10 peaks in the section of the pulse waveform W2, and the irregular pulse.

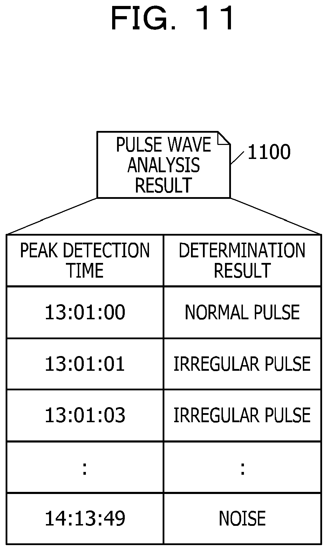

In this manner, by displacing the sections from each other by one peak, the user may identify which of "normal pulse", "irregular pulse", and "noise" the last peak included in the pulse waveform Wj indicates. For this reason, the output unit 805 may output a pulse wave analysis result 1100 as illustrated in FIG. 11.

FIG. 11 illustrates a specific example (2) of the pulse wave analysis result. In FIG. 11, in the pulse wave analysis result 1100, the peak detection time is associated with the determination result. According to the pulse wave analysis result 1100, the user may determine which of "normal pulse", "irregular pulse", and "noise" each peak indicates. The peak detection time indicates the time when the peak is detected (hour: minute: second).

For example, the user may recognize that the peak detected at the peak detection time "13:01:00" represents "normal pulse". The user may recognize that the peak detected at the peak detection time "13:01:01" represents "irregular pulse". The user may recognize that the peak detected at the peak detection time "14:13:49" represents "noise".

(Use of pulse wave area $S_i$ in place of amplitude $Amp_i$)

In the above description, the matching score of the pulse waveform Wj is calculated based on the pair of the amplitude $Amp_i$ and the pulse wave interval $RRI_i$. However, the present disclosure is not limited to this. For example, the calculation unit 803 may use the pulse wave area $S_i$ in place of the amplitude $Amp_i$ to calculate the matching score of the pulse waveform Wj, based on the pair of the pulse wave area $S_i$ and the pulse wave interval $RRI_i$.

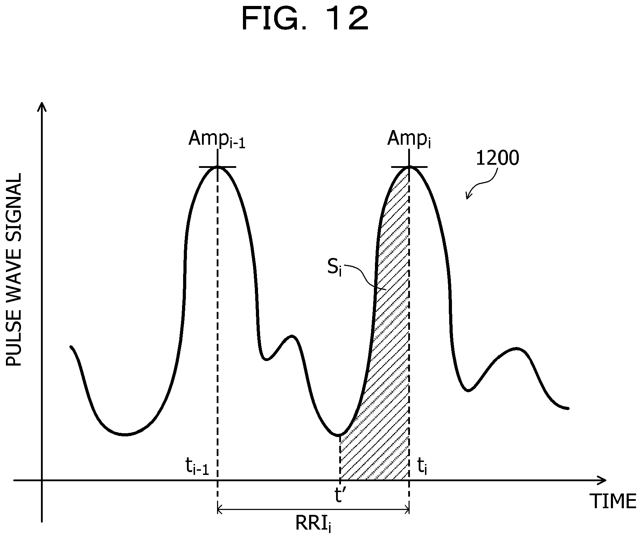

Here, referring to FIG. 12, a specific example of the pulse wave area $S_i$ will be described.

FIG. 12 illustrates a specific example of the pulse wave area. In FIG. 12, a pulse waveform 1200 is the waveform indicating the temporal change in pulse waves. For example, the pulse wave area $S_i$ may be defined as an area (in FIG. 12, a region expressed by inclined lines) from the smallest value (minimum value) to the largest value (maximum value) of the pulse wave signal between pulse waves.

In this case, the calculation unit 803 may calculate the pulse wave area $S_i$ by applying a definite integral to the section from a detection time t' of the smallest value (minimum value) to a peak detection time $t_i$ in the pulse waveform 1200. That is, the pulse wave area $S_i$ is proportional to the amplitude $Amp_i$, and may be used in the same manner as the amplitude $Amp_i$. In the case of using the pulse wave area $S_i$ in place of the amplitude $Amp_i$, "Amp" included in the equation (1) becomes "S".

(Decision of threshold Th and predetermined range R2)

Next, an example of decision of the threshold Th and the predetermined range R2 will be described. In the above description, the threshold Th and the predetermined range R2 are common to all subjects. However, the present disclosure is not limited to this. For example, since a person having a weak pulse signal has a small determination coefficient (matching score) and a small b value, it may be determined that the pulse waveform contains noise at all times.

For this reason, first, it is determined that the subject's pulse waveform measured in rest is classified as "normal pulse", "irregular pulse", or "noise" indicates. Then, when the pulse waveform has been determined as "noise", but has never determined as "irregular pulse", the threshold Th and the predetermined range R2 may be changed.

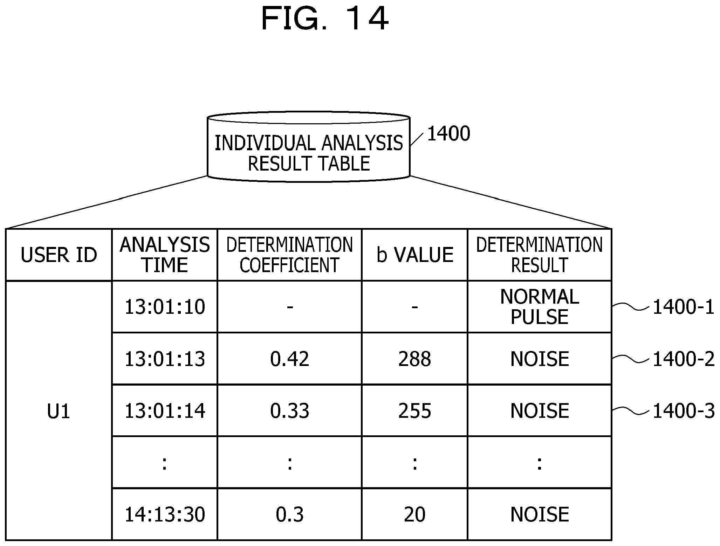

The pulse waveform measured in rest is stored in, for example, the individual pulse waveform table 1300 in individual characteristic DB 340 of the server 302 (See FIG. 13). The determination result for the pulse waveform measured in rest is stored in, for example, the individual analysis result table 1400 in the individual characteristic DB 340 of the server 302 (See FIG. 14).

FIG. 13 illustrates an example of contents stored in the individual pulse waveform table 1300. In FIG. 13, the individual pulse waveform table 1300 has fields for the user ID, the peak detection time, the amplitude, and the pulse wave interval, sets information to each of the fields, thereby storing individual pulse wave information (for example, individual pulse wave information 1300-1 to 1300-3) as records.

Here, the user ID is an identifier that uniquely identifies the subject. The peak detection time is a time when the peak of the pulse wave is detected. The amplitude is a difference between the maximum value (peak) and the minimum value of the pulse wave signal. The pulse wave interval is an interval between consecutive peaks in time series.

The pulse wave analysis apparatus 301 may access the server 302 to refer to the individual pulse waveform table 1300. Then, the pulse wave analysis apparatus 301 may classify the subject's pulse waveform measured in rest as "normal pulse", "irregular pulse", or "noise".

The obtained determination result is stored in, for example, the individual analysis result table 1400. The pulse wave analysis apparatus 301 may have the individual pulse waveform table 1300.

FIG. 14 illustrates an example of contents stored in the individual analysis result table 1400. In FIG. 14, the individual analysis result table 1400 ha fields for the user ID, the analysis time, the determination coefficient, the b value, and the determination result, and sets information to each of the fields, thereby storing individual analysis results (for example, individual analysis results 1400-1 to 1400-3) as records.

Here, the user ID is an identifier that uniquely identifies the subject. The analysis time is a time when the pulse waveform to be analyzed is analyzed. The determination coefficient is a value indicating the matching score of the pulse waveform to be analyzed. The b value is a the coefficient value b included in the equation (1). The determination result indicates that the pulse waveform to be analyzed is classified as "normal pulse", "irregular pulse", and "noise".

The pulse wave analysis apparatus 301 may access the server 302 to refer to the individual analysis result table 1400. The pulse wave analysis apparatus 301 may have the individual analysis result table 1400.

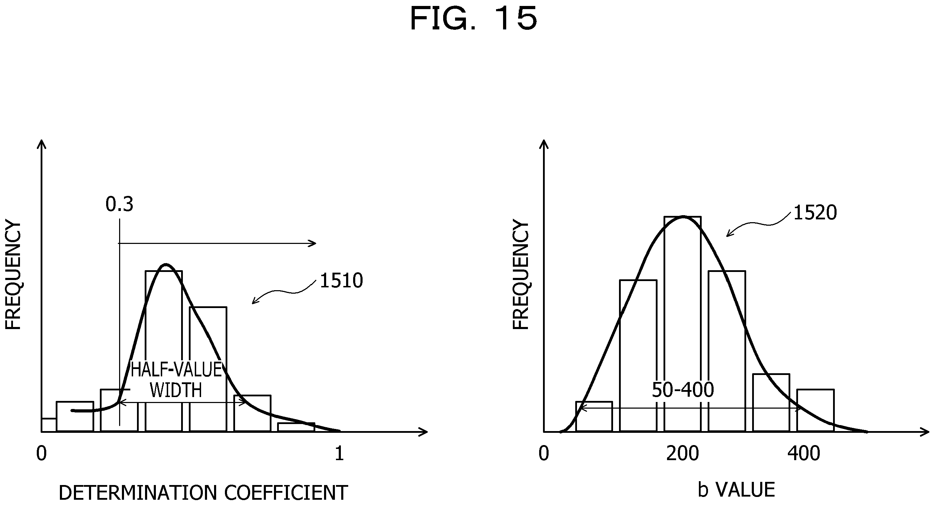

Referring to FIG. 15, an example of decision of the threshold Th and the predetermined range R2 will be described.

FIG. 15 illustrates the example of decision of the threshold Th and the predetermined range R2. The pulse wave analysis apparatus 301 refers to the individual analysis result table 1400 to create a frequency distribution 1510 of the determination coefficient. The frequency distribution 1510 indicates how frequently the determination coefficient of each value appears.

Next, the pulse wave analysis apparatus 301 calculates a half-value width of the determination coefficient, based on the frequency distribution 1510. The half-value width is an indicator indicating how wide a mountain-shaped function is, and is a width of the mountain at a half height of the mountain peak. Here, it is assumed that "0.3 to 0.7" is calculated as the half-value width of the determination coefficient. In this case, for example, the pulse wave analysis apparatus 301 decides the lower limit "0.3" of the half-value width of the determination coefficient, as the threshold Th.

For example, the pulse wave analysis apparatus 301 refers to the individual analysis result table 1400 to create a frequency distribution 1520 of the b value. The frequency distribution 1520 indicates how frequently each value appears as the b value. Next, the pulse wave analysis apparatus 301 calculates the half-value width of the b value, based on the frequency distribution 1520. Here, it is assumed that "50 to 400" is calculated as the half-value width of the b value. In this case, for example, the pulse wave analysis apparatus 301 decides the half-value width of the b value "50 to 400", as the predetermined range R2.

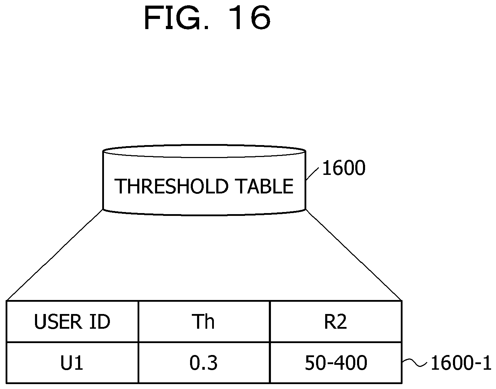

For example, the decided threshold Th and predetermined range R2 are associated with the user ID of the subject, and are stored in the threshold table 1600 illustrated in FIG. 16.

FIG. 16 illustrates an example of contents stored in the threshold table 1600. In FIG. 16, the threshold table 1600 sets fields for the user ID, Th, and R2, and sets information to each of the fields, thereby storing threshold information (for example, threshold information 1600-1) as records.

Here, the user ID is an identifier that uniquely identifies the subject. Th is a value of the threshold Th. R2 is a range of the predetermined range R2. According to the threshold table 1600, the threshold Th and the predetermined range R2 that are used to analyze the pulse waves of the subject having the user ID "U1" may be identified.

The above-described functional units of the pulse wave analysis apparatus 301 may be embodied by the server 302 illustrated in FIG. 3. In this case, using the pulse wave analysis apparatus 301, the user may refer to the pulse wave analysis result (for example, the pulse wave analysis results 1000, 1100) outputted from the server 302.

(Pulse wave analysis processing procedure of pulse wave analysis apparatus 301)

Next, a pulse wave analysis processing procedure of the pulse wave analysis apparatus 301 in accordance with Embodiment 1 will be described.

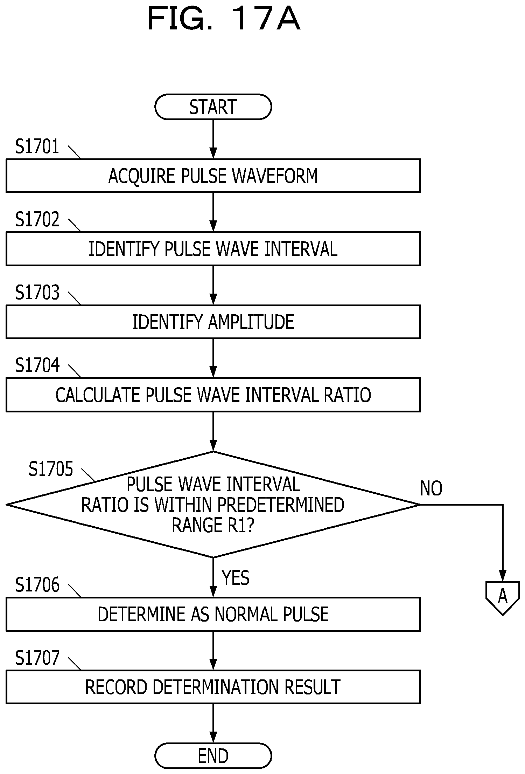

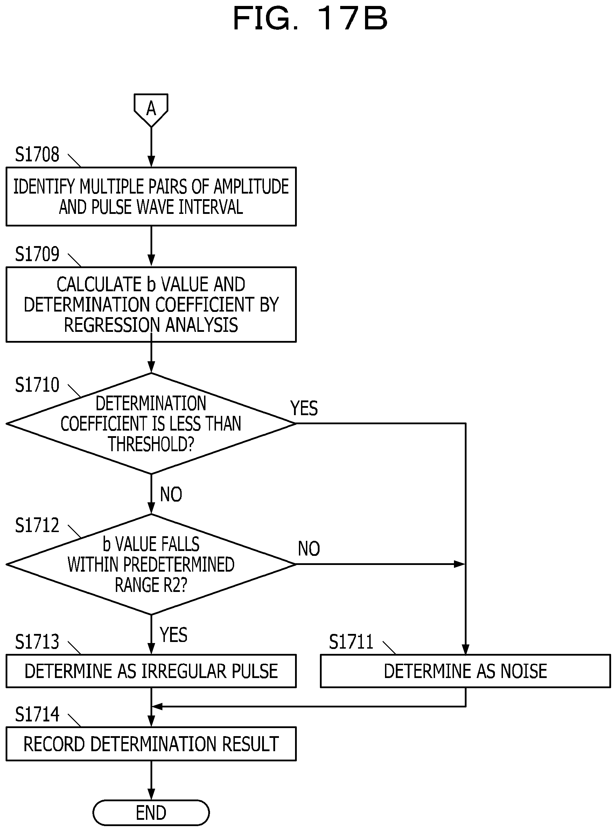

FIGs. 17A and 17B are flow charts illustrating examples of the pulse wave analysis processing procedure of the pulse wave analysis apparatus 301 in accordance with Embodiment 1. In the flow chart in FIG. 17A, first, the pulse wave analysis apparatus 301 acquires the pulse waveform Wj (Step S1701).

Next, the pulse wave analysis apparatus 301 identifies the pulse wave intervals $RRI_1$ to $RRI_n$ of the pulse waves, based on the acquired pulse waveform Wj (Step S1702). The pulse wave analysis apparatus 301 identifies the pulse wave amplitudes $Amp_1$ to $Amp_n$, based on the acquired pulse waveform Wj (Step S1703).

The identified pulse wave intervals $RRI_1$ to $RRI_n$ and amplitudes $Amp_1$ to $Amp_n$ are stored in, for example, the pulse waveform DB 320.

Next, the pulse wave analysis apparatus 301 refers to the pulse waveform DB 320 to calculate the pulse wave interval ratio ($RRI_{i+1}/RRI_i$) between adjacent pulse waves in the pulse waveform Wj (Step S1704). Then, the pulse wave analysis apparatus 301 determines whether or not the calculated pulse wave interval ratio ($RRI_{i+1}/RRI_i$) falls within the predetermined range R1 (Step S1705).

Here, when the pulse wave interval ratio ($RRI_{i+1}/PPI_i$) falls within the predetermined range R1 (Step S1705: Yes), pulse wave analysis apparatus 301 determines that the pulse waveform Wj indicates the normal pulse (Step S1706). Then, the pulse wave analysis apparatus 301 records the obtained determination result in the analysis result DB 330 (Step S1707), and terminates the series of processing in this flow chart.

In Step S1705, when the pulse wave interval ratio ($RRI_{i+1}/RRI_i$) falls outside the predetermined range R1 (Step S1705: No), the pulse wave analysis apparatus 301 proceeds to Step S1708 illustrated in FIG. 17B.

In the flow chart in FIG. 17B, first, the pulse wave analysis apparatus 301 refers to the pulse waveform DB 320 to identify multiple pairs of the pulse wave amplitude $Amp_i$ and the pulse wave interval $RRI_i$ in the pulse waveform Wj (Step S1708).

Next, the pulse wave analysis apparatus 301 performs regression analysis based on the multiple identified pairs P1 to Pn using the above equation (1) to calculate the b value and the determination coefficient (Step S1709). Then, the pulse wave analysis apparatus 301 determines whether or not the calculated determination coefficient is less than the threshold Th (Step S1710).

Here, when the determination coefficient is less than the threshold Th (Step S1710: Yes), the pulse wave analysis apparatus 301 determines that the pulse waveform Wj contains noise (Step S1711), and proceeds to Step S1714.

On the other hand, when the determination coefficient is equal or greater than the threshold Th (Step S1710: No), the pulse wave analysis apparatus 301 determines whether or not the calculated b value falls within the predetermined range R2 (Step S1712). Here, when the b value falls outside the predetermined range R2 (Step S1712: No), the pulse wave analysis apparatus 301 proceeds to Step S1711.

On the other hand, when the b value falls within the predetermined range R2 (Step S1712: Yes), the pulse wave analysis apparatus 301 determines that the pulse waveform Wj includes the irregular pulse (Step S1713). Then, the pulse wave analysis apparatus 301 records the obtained determination result in the analysis result DB 330 (Step S1714), and terminates the series of processing in this flow chart.

Thereby, the pulse waveform Wk may be accurately classified as "normal pulse", "irregular pulse", or "noise".

When the threshold Th unique to the subject is stored in the threshold table 1600 of the server 302, in Step S1709, the pulse wave analysis apparatus 301 uses the threshold Th unique to the subject in the threshold table 1600.

When the predetermined range R2 unique to the subject is stored in the threshold table 1600 of the server 302, in Step S1710, the pulse wave analysis apparatus 301 uses the predetermined range R2 unique to the subject is stored in the threshold table 1600.

When the determination coefficient is less than the threshold Th in Step S1710 (Step S1710: Yes), the pulse wave analysis apparatus 301 may delete the pair Pi as an outlier from the pairs P1 to Pn. Then, when the number of pairs after deletion is three or more, the pulse wave analysis apparatus 301 may return to Step S1704, and repeat the series of processing. On the other hand, when the number of pairs after deletion is less than three, the pulse wave analysis apparatus 301 determines that the pulse waveform Wj contains noise.

Similarly, when the b value falls within the predetermined range R2 in Step S1712 (Step S1712: No), the pulse wave analysis apparatus 301 may delete the pair Pi as the outlier from the pairs P1 to Pn. Then, when the number of pairs after deletion is three or more, the pulse wave analysis apparatus 301 may return to Step S1704 and repeat the series of processing. On the other hand, when the number of pairs after deletion is less than three, the pulse wave analysis apparatus 301 determines that the pulse waveform Wj contains noise.

Thereby, the outlier may be removed from data used in regression analysis and retry pulse wave analysis, improving the accuracy of pulse wave analysis.

As has been described, the pulse wave analysis apparatus 301 in accordance with Embodiment 1 may identify the pulse wave intervals $RRI_i$ of pulse waves, based on the acquired pulse waveform Wj. The pulse wave analysis apparatus 301 may calculate the pulse wave interval variation score based on the identified pulse wave intervals $RRI_i$. The pulse wave analysis apparatus 301 may determine whether or not the pulse waveform Wj indicates the normal pulse based on the calculated pulse wave interval variation score.

Thereby, the pulse waveform Wj having a small variation degree between pulse wave intervals may be classified as "normal pulse".

The pulse wave analysis apparatus 301 may identify the pulse wave amplitude $Amp_i$, based on the acquired pulse waveform Wj. The pulse wave analysis apparatus 301 may perform regression analysis based on the pairs P1 to Pn using the equation (1) to calculate values of the coefficients a, b and the determination coefficient. The pairs P1 to Pn are pairs of the amplitude $Amp_1$ to $Amp_n$ and each of the pulse wave intervals $RRI_1$ to $RRI_n$.

This may find the determination coefficient indicating the matching degree between the pulse waveform Wj and the model expressed as the function F representing the temporal change in the blood flow volume with heartbeat (contraction and relaxation), and the b value (the coefficient value b) corresponding to the refractory period of the heart.

The pulse wave analysis apparatus 301 may determine that the pulse waveform Wj contains noise, when the calculated determination coefficient (matching score) is less than the threshold Th. Thereby, the pulse waveform Wj having a low matching degree with the model representing the temporal change in the blood flow volume with heartbeat may be classified as "noise".

The pulse wave analysis apparatus 301 may determine that the pulse waveform Wj includes the irregular pulse, when it is determined that the pulse waveform Wj does not indicate the normal pulse, and the calculated determination coefficient is equal or greater than the threshold Th. Thereby, the pulse waveform Wj having a large variation degree between pulse wave intervals and a high matching degree with the model representing the temporal change in the blood flow volume with heartbeat may be classified as "irregular pulse".

The pulse wave analysis apparatus 301 may determine that the pulse waveform Wj contains noise, when the calculated b value (the coefficient value b) falls outside the predetermined range R2. Thereby, the pulse waveform Wj in which the b value corresponding to the refractory period of the heart falls outside the predetermined range R2 representing the general refractory period may be classified as "irregular pulse".

The pulse wave analysis apparatus 301 may delete the pair Pi as the outlier from the pairs P1 to Pn, when the determination coefficient of the calculated pulse waveform Wj is less than the threshold Th, or the calculated b value falls outside the predetermined range R2. This may remove a pair that greatly deviates from the general tendency of data from the pairs P1 to Pn.

The pulse wave analysis apparatus 301 may delete the pair Pi as the outlier from the pairs P1 to Pn and then, recalculate the pulse wave interval variation score in the deleted pair. This may recalculate the variation score based on data with the outlier removed, improving the accuracy of pulse wave analysis.

Using the above equation (1), the pulse wave analysis apparatus 301 may delete the pair Pi as the outlier from the pairs P1 to Pn and then, perform regression analysis based on the pairs with the pair Pi deleted to recalculate values of the coefficients a, b and the determination coefficient. This may recalculate the values of the coefficients a, b and the determination coefficient, based on the data with the outlier removed, improving the accuracy of pulse wave analysis.

Therefore, the pulse wave analysis apparatus 301 in accordance with Embodiment 1 may accurately classify the pulse waveform Wk as "normal pulse", "irregular pulse" or "noise". This may separate noise caused by the body motion and so on from the subject's pulse waves, improving the accuracy of analyzing pulse waves such as irregular pulse determination and pulse rate determination.

For example, pulse waves may be subconsciously measured by applying the pulse wave analysis apparatus 301 to a wearable terminal capable of measuring pulse waves through the use of face pulse measurement. This may measure pulse waves waiting time in hospitals, free time at home, and the like, early finding an irregular pulse to contribute to avoidance of severe diseases including heart failure and cerebral infarction.

(Embodiment 2)

Next, a pulse wave analysis apparatus 301 in accordance with Embodiment 2 will be described. The same elements as those in Embodiment 1 are not illustrated and described.

In Embodiment 1, using the pulse waveform of pulse waves measured in the section of about 10 seconds, the pulse waveform is classified as "normal pulse", "irregular pulse", or "noise". However, in measuring pulse waves by face pulse measurement, it may be impossible to acquire one section of the pulse waveform for about 10 seconds because of the difficulty in continuously taking an image of the subject's face for about 10 seconds.

For this reason, in Embodiment 2, description is provided for a determination method of combining pulse waveforms of pulse waves measured in different sections and determining the pulse waveforms as "normal pulse", "irregular pulse", or "noise". However, when pulse waves are measured in different sections, the state of the subject's heart, measurement environment, and so on may vary between the sections. In this case, the pulse wave amplitudes measured in the sections are different from each other in magnitude, and therefore the regression analysis simply using the pair of pulse wave interval and amplitude (or pulse wave area) may result in lower accuracy of the pulse wave analysis.

Thus, to absorb the difference between the amplitudes in magnitude due to the variations in the state of the heart, measurement environment and so on at measurement, Embodiment 2 uses the adjacent pulse wave amplitude ratio (or area ratio) in each section instead of simply using the pulse wave amplitude (or pulse wave area), and thereby suppresses a reduction in the accuracy of pulse wave analysis.

(Functional configuration of pulse wave analysis apparatus 301)

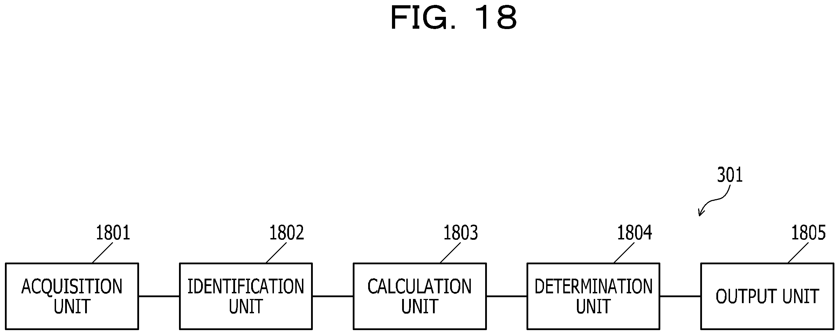

FIG. 18 is a block diagram illustrating an example of functional configuration of a pulse wave analysis apparatus 301 in accordance with Embodiment 2. In FIG. 18, the pulse wave analysis apparatus 301 includes an acquisition unit 1801, an identification unit 1802, a calculation unit 1803, a determination unit 1804, and an output unit 1805. the acquisition unit 1801 to output unit 1805 function as control units, and specifically, cause the CPU 401 to execute the programs stored in the storage devices such as the memory 402 and the disk 404 in FIG. 4, or use the I/F 405 to achieve their functions. Processing results of the function units are stored in the storage devices such as the memory 402 and the disk 404.

The acquisition unit 1801 acquires pulse waveforms having a temporal change in pulse waves measured in different sections. The sections may be continuous in time series, or discontinuous in time series. The time width of each section may be set to any value, and different sections may have different time width. However, each section has to have at least three peaks of pulse waves.

In the following description, different sections may be referred to as "sections S1 to SK". K is a natural number of 3 or more, such as 10. Any section among the sections S1 to SK may be referred to as "section Sk" (k = 1, 2, ..., K). The combined pulse waveform in the sections S1 to SK may be referred to as "pulse waveform W".

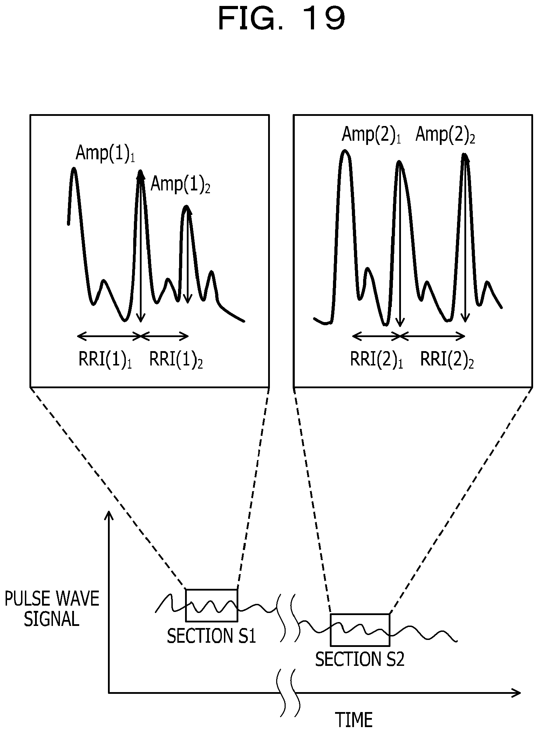

Here, referring to FIG. 19, a specific example of different sections will be described.

FIG. 19 illustrates the temporal change in the pulse waveform. In FIG. 19, the sections S1, S2 are different sections in which the subject's pulse waves are measured. The sections S1, S2 are discontinuous sections in time series. The sections S1, S2 each include three peaks of pulse waves.

Returning description with reference to FIG. 18, the identification unit 1802 identifies a combination of the wave amplitude ratio between adjacent pulses and the pulse wave intervals from the pulse waveform acquired in each section Sk.

Using the sections S1, S2 illustrated in FIG. 19, first, the identification unit 1802 identifies pulse wave intervals $RRI(1)_1$, $RRI(1)_2$ between adjacent pulse waves in the section S1. Next, the identification unit 1802 identifies amplitudes $Amp(1)_1$, $Amp(1)_2$ of the adjacent pulse waves in the section S1. Then, the identification unit 1802 calculates the amplitude ratio "$AmpRatio(1) = Amp(1)_2/Amp(1)_1$" between the adjacent pulse waves in the section S1.

In this way, the identification unit 1802 identifies the combination of the amplitude ratio "$AmpRatio(1) = Amp(1)_2/Amp(1)_1$" between the adjacent pulse waves and the pulse wave intervals $RRI(1)_1$, $RRI(1)_2$ in the section S1.

Similarly, the identification unit 1802 identifies pulse wave intervals $RRI(2)_1$, $RRI(2)_2$ between adjacent pulse waves in the section S2. Next, the identification unit 1802 identifies amplitudes $Amp(2)_1$, $Amp(2)_2$ of the adjacent pulse waves in the section S2. Then, the identification unit 1802 calculates the amplitude ratio "$AmpRatio(2) = Amp(2)_2/Amp(2)_1$" between the adjacent pulse waves in the section S2.

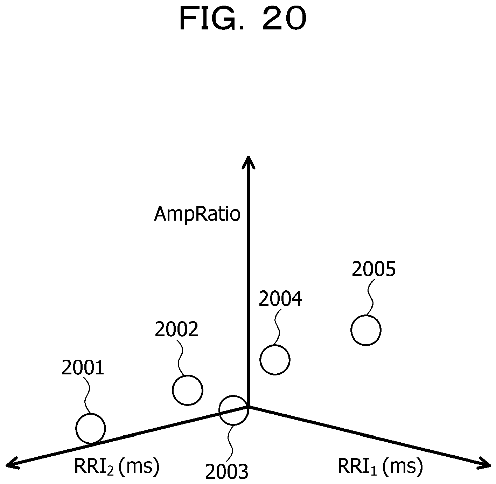

Thus, the identification unit 1802 identifies the combination of the amplitude ratio "$AmpRatio(2) = Amp(2)_2/Amp(2)_1$" between the adjacent pulse waves and the pulse wave intervals $RRI(2)_1$, $RRI(2)_2$ in the section S2. An example of the relationship between the amplitude ratio ($AmpRatio(k)$) between the adjacent pulse waves and the pulse wave intervals $RRI(k)_1$, $RRI(k)_2$ in each section Sk is illustrated in FIG. 20.

FIG. 20 illustrates the relationship between the amplitude ratio between adjacent pulse waves and the pulse wave intervals in each section Sk. The example in FIG. 20 illustrates a three-dimensional coordinate system in which data pieces 2001 to 2005 for the sections S1 to S5 are presented, the data pieces 2001 to 2005 each indicating a correspondence between the amplitude ratio AmpRatio between adjacent pulse waves and the pulse wave intervals $RRI_1$, $RRI_2$ in the corresponding one of the sections S1 to S5.

In the following description, the combination of the amplitude ratio "$AmpRatio(k) = Amp(k)_2/Amp(k)_1$" between adjacent pulse waves and the pulse wave intervals $RRI(k)_1$, $RRI(k)_2$ in the section Sk may be referred to as "combination Ck".

Returning to the description with reference to FIG. 18, the calculation unit 1803 calculates the variation score between identified pulse wave intervals. Specifically, for example, the calculation unit 1803 may calculate the pulse wave interval ratio ($RRI(k)_2/RRI(k)_1$) between adjacent pulse waves in each section Sk as the pulse wave interval variation score.

The determination unit 1804 determines whether or not the pulse waveform W indicates the normal pulse, based on the calculated pulse wave interval variation score. Specifically, for example, the determination unit 1804 may determine that the pulse waveform W indicates the normal pulse, when the pulse wave interval variation score falls within a predetermined range R1. On the other hand, the determination unit 1804 may determine that the pulse waveform W does not indicate the normal pulse, when the pulse wave interval variation score falls outside the predetermined range R1.

More specifically, for example, the determination unit 1804 may determine that the pulse waveform W indicates the normal pulse, when all the pulse wave interval ratios $(RRI(k)_2/RRI(k)_1)$ in each section Sk included in the pulse waveform W fall within the predetermined range R1. The determination unit 1804 may determine that the pulse waveform W indicates the normal pulse, for example, when a predetermined ratio (for example, 90%) or more of the pulse wave interval ratios in the entire section in the pulse waveform W falls within the predetermined range R1.

This makes it possible to classify the pulse waveform W having a small variation degree between pulse wave intervals as "normal pulse".

The calculation unit 1803 calculates the matching score between the pulse waveform W and the model expressed as the function F. Here, as previously described, the function F is a function that indicates the temporal change in the blood flow volume with heartbeat (contraction and relaxation). For example, the following equation (3) may be used as the function F. Here, AmpRatio denotes an amplitude ratio between adjacent pulse waves in each section, Amp denotes the pulse wave amplitude, RRI denotes the pulse wave interval, and a, b are coefficients.

[Equation 3]

$$AmpRatio = \frac{Amp_2}{Amp_1} = \frac{\sqrt{a*(RRI_2 - b)}}{\sqrt{a*(RRI_1 - b)}} \quad \cdots (3)$$

Specifically, for example, first, when it is determined that the pulse waveform W does not indicate the normal pulse, the calculation unit 1803 performs regression analysis based on the identified combinations C1 to CK using the above equation (3) to calculate the b value (the coefficient value b) and the determination coefficient. The determination coefficient corresponds to the matching score of the pulse waveform W.

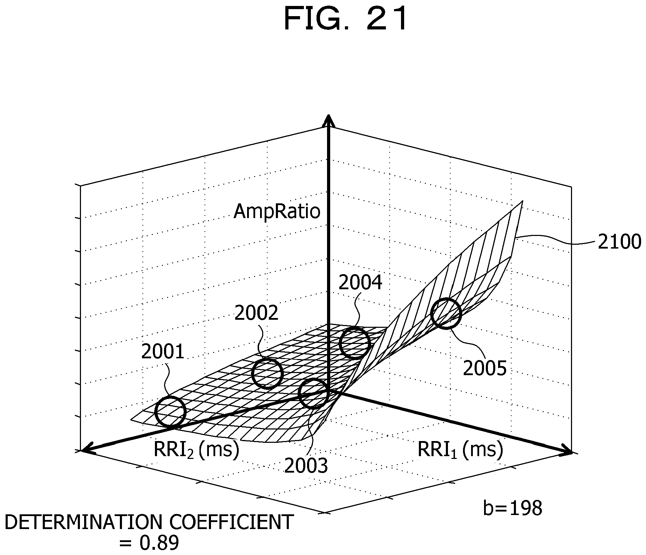

Specifically, the determination coefficient denotes how much the relationship between the values of the pulse wave intervals $RRI(k)_1$, $RRI(k)_2$ minus the b value and the pulse wave amplitude ratio AmpRatio(k) in each section Sk matches with the model expressed by the ratio of a function which increases monotonously in a convex upward shape while passing the origin. This regression analysis, for example, figures out an approximate curve as illustrated in FIG. 21.

FIG. 21 illustrates a specific example of an approximate plane. In FIG. 21, an approximate plane 2100 is an approximate plane found by regression analysis based on the data pieces 2001 to 2005 illustrated in FIG. 20 (regression equation). In the example illustrated in FIG. 21, the determination coefficient is "0.89", and the b value is "198".

Returning to the description with reference to FIG. 18, the determination unit 1804 determines whether or not the pulse waveform W contains noise, based on the calculated matching score. Specifically, for example, the determination unit 1804 may determine that the pulse waveform W contains noise, when the calculated matching score is less than the threshold Th.

Thereby, the pulse waveform W having a low matching degree with the model representing the temporal change in the blood flow volume with heartbeat may be classified as "noise".

For example, the determination unit 1804 may determine that the pulse waveform W includes the irregular pulse, when it is determined that the pulse waveform W does not indicate the normal pulse, and the calculated matching score is equal or greater than the threshold Th. Thereby, the pulse waveform W having a high matching degree with the model representing the temporal change in the blood flow volume with heartbeat may be classified as "irregular pulse".

The determination unit 1804 may determine that the pulse waveform W contains noise, when the calculated b value falls outside the predetermined range R2. Thereby, the pulse waveform W having the b value corresponding to the refractory period of the heart outside the predetermined range R2 representing the general refractory period may be classified as "irregular pulse".

The calculation unit 1803 may delete the combination Ck as the outlier from the combinations C1 to CK, when the matching score of the calculated pulse waveform W is less than the threshold Th, or the calculated b value falls outside the predetermined range R2.

Specifically, for example, the calculation unit 1803 may compare each of the combinations C1 to CK with the approximate plane (regression equation) found by regression analysis, and delete the combination that deviates the most as the outlier. For example, the calculation unit 1803 may delete data that deviates from an average of sample values by three times of a standard deviation or more, as the outlier.

Then, the calculation unit 1803 may delete the combination Ck as the outlier from the combinations C1 to CK, and perform regression analysis based on the combinations including no deleted combination Ck using the above equation (3), to recalculate the b value and the determination coefficient. Thereby, the b value and the determination coefficient may be recalculated based on the data without the outlier, improving the accuracy of pulse wave analysis.

However, to perform regression analysis, at least three pieces of data are requested. For this reason, when the number of combinations before deletion of the combination as the outlier is four or more, the b value and the determination coefficient may be recalculated.

The output unit 1805 outputs the obtained determination result. Specifically, for example, the output unit 1805 may output the pulse wave analysis result associating the analysis time T of the pulse waveform W with the determination result of the pulse waveform W. For example, information identifying the measurement period of each section Sk may be outputted as the analysis time T.

The peak detection time of the peak in each section Sk may be outputted as the analysis time T. At this time, the peak detection time of the peak corresponding to the combination deleted as the outlier is not outputted. For this reason, for example, when it is determined that the pulse waveform W contains the irregular pulse, the user may determine that the undeleted peak corresponding to remaining peak detection time is the irregular pulse.

In the above description, the matching score of the pulse waveform W is calculated based on the combination Ck of the amplitude ratio between adjacent pulse waves and the pulse wave intervals $RRI(k)_1$, $RRI(k)_2$ in the section Sk. However, the present disclosure is not limited to this. For example, the calculation unit 1803 may use the area ratio between adjacent pulse waves (ratio between the pulse wave areas) in the section Sk in place of the amplitude ratio, and calculate the matching score of the pulse waveform W, based on the combination Ck of the area ratio and the pulse wave intervals $RRI(k)_1$, $RRI(k)_2$. In the case of using the area ratio in place of the amplitude ratio, for example, "S (pulse wave area)" is substituted for "Amp" included in the equation (3).

(Pulse wave analysis processing procedure of pulse wave analysis apparatus 301)

Next, a pulse wave analysis processing procedure of the pulse wave analysis apparatus 301 in accordance with Embodiment 2 will be described below.

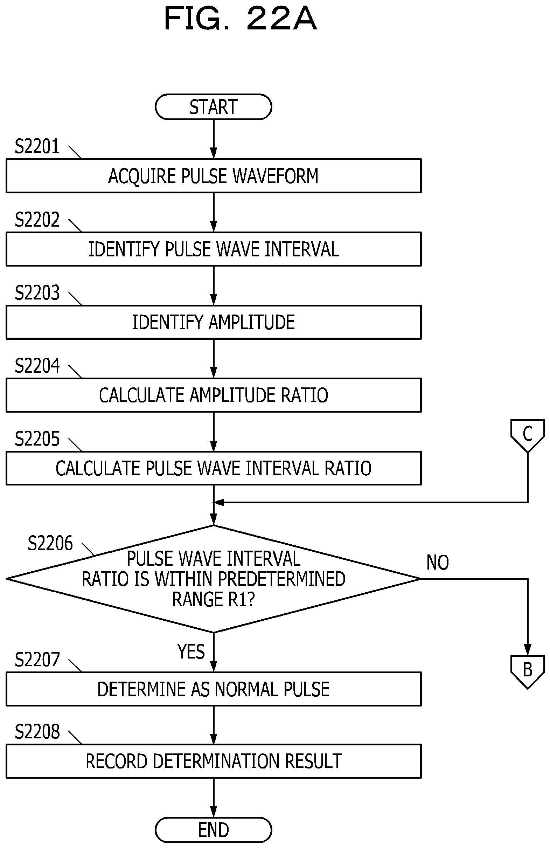

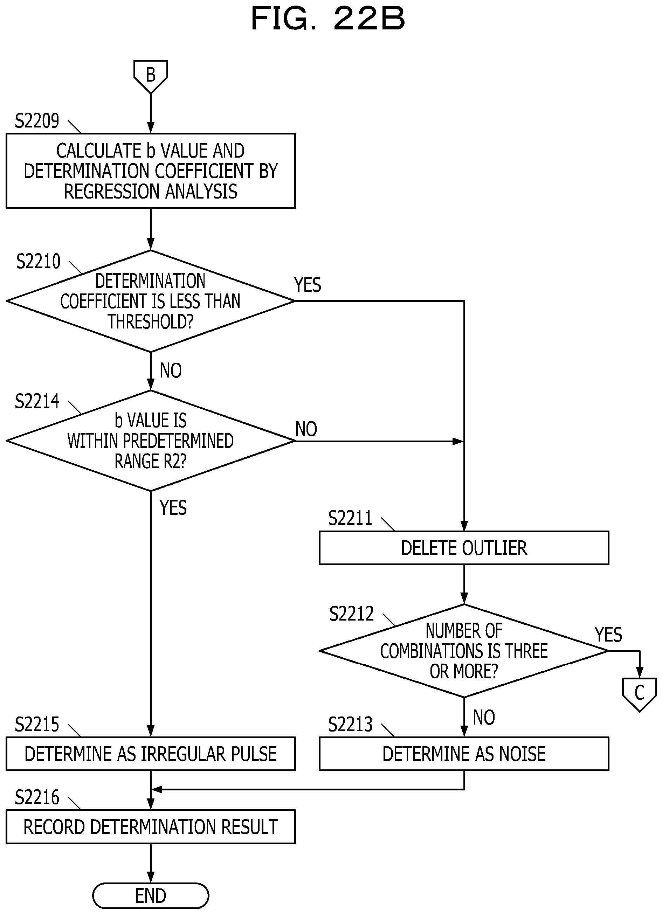

FIGs. 22A and 22B are flow charts illustrating an example of pulse wave analysis processing procedure of pulse wave analysis apparatus 301 in accordance with Embodiment 2. In the flow chart in FIG. 21, first, the pulse wave analysis apparatus 301 acquires the pulse waveform indicating the temporal change in pulse waves measured in each of the sections S1 to SK (Step S2201).

Next, the pulse wave analysis apparatus 301 identifies the pulse wave intervals $RRI(k)_1$, $RRI(k)_2$ of adjacent pulse waves in each section Sk, based on the acquired pulse waveform in each of the sections S1 to SK (Step S2202). The pulse wave analysis apparatus 301 identifies the amplitudes $Amp(k)_1$, $Amp(k)_2$ of the adjacent pulse waves in the section Sk, based on the acquired pulse waveform in each of the sections S1 to SK (Step S2203).

Then, the pulse wave analysis apparatus 301 calculates the amplitude ratio $AmpRatio(k)$ (= $Amp(k)_2/Amp(k)_1$) between the adjacent pulse waves in the section Sk (Step S2204). The pulse wave analysis apparatus 301 calculates the pulse wave interval ratio ($RRI(k)_{i+1}/RRI(k)_i$) between the adjacent pulse waves in the section Sk (Step S2205).

Then, pulse wave analysis apparatus 301 determines whether or not the calculated pulse wave interval ratio ($RRI(k)_{i+1}/RRI(k)_i$) in the section Sk falls within the predetermined range R1 (Step S2206). Here, when the pulse wave interval ratio ($RRI(k)_{i+1}/RRI(k)_i$) falls within the predetermined range R1 (Step S2206: Yes), the pulse wave analysis apparatus 301 determines that the pulse waveform W indicates the normal pulse (Step S2207).

Then, the pulse wave analysis apparatus 301 records the obtained determination result in the analysis result DB 330 (Step S2208), and terminates the series of processing in this flow chart. The pulse waveform W is acquired by combining the pulse waveforms in the sections S1 to SK.

When the pulse wave interval ratio ($RRI(k)_{i+1}/RRI(k)_i$) falls outside the predetermined range R1 in Step S2206 (Step S2206: No), the pulse wave analysis apparatus 301 proceeds to Step S2209 illustrated in FIG. 22B.

In the flow chart in FIG. 22B, first, the pulse wave analysis apparatus 301 performs regression analysis based on the combination Ck in the section Sk using the above equation (3) to calculate the b value and the determination coefficient (Step S2209). The combination Ck is a combination of the amplitude ratio $AmpRatio(k)$ between adjacent pulse waves and the pulse wave intervals $RRI(k)_1$, $RRI(k)_2$ in the section Sk.

When the outlier is deleted in below-mentioned Step S2211, the pulse wave analysis apparatus 301 performs regression analysis based on the combination Ck in the section Sk with of the outlier deleted.

Then, the pulse wave analysis apparatus 301 determines whether or not the calculated determination coefficient is less than the threshold Th (Step S2210). Here, when the determination coefficient is less than the threshold Th (Step S2210: Yes), the pulse wave analysis apparatus 301 deletes the combination as the outlier from the combinations Ck in the sections Sk (Step S2211).

Then, the pulse wave analysis apparatus 301 determines whether or not the number of combinations in the section Sk after deletion of the outlier is three or more (Step S2212). Here, when the number of combinations in the section Sk after deletion is less than three (Step S2212: No), the pulse wave analysis apparatus 301 determines that the pulse waveform W contains noise (Step S2213), and proceeds to Step S2216.

On the other hand, when the number of combinations in the section Sk after deletion is three or more (Step S2212: Yes), the pulse wave analysis apparatus 301 returns to Step S2206 illustrated in FIG. 22A. Then, the pulse wave analysis apparatus 301 determines whether or not the pulse wave interval ratio ($RRI(k)_{i+1}/RRI(k)_i$) in the section Sk with the outlier deleted falls within the predetermined range R1.

When the determination coefficient is equal or greater than the threshold Th in Step S2210 (Step S2210: No), the pulse wave analysis apparatus 301 determines whether or not the calculated b value falls within a predetermined range R2 (Step S2214). Here, when the b value falls outside the predetermined range R2 (Step S2214: No), the pulse wave analysis apparatus 301 proceeds to Step S2211.

On the other hand, when the b value falls within the predetermined range R2 (Step S2214: Yes), the pulse wave analysis apparatus 301 determines that the pulse waveform W includes the irregular pulse (Step S2215). Then, the pulse wave analysis apparatus 301 records the obtained determination result in the analysis result DB 330 (Step S2216), and terminates the series of processing in this flow chart.

Thereby, the pulse waveform W acquired by combining pulse waves measured in the sections S1 to SK may be accurately classified as "normal pulse", "irregular pulse", or "noise".

As has been described, the pulse wave analysis apparatus 301 in accordance with Embodiment 2 may identify the pulse wave intervals $RRI(k)_1$, $RRI(k)_2$ of adjacent pulse waves in the section Sk based on the pulse waveform in the section Sk. The pulse wave analysis apparatus 301 may calculate the pulse wave interval variation score, based on the identified pulse wave intervals $RRI(k)_1$, $RRI(k)_2$. Then, the pulse wave analysis apparatus 301 may determine whether or not the pulse waveform W indicates the normal pulse, based on the calculated pulse wave interval variation score.

Thereby, the pulse waveform W having a small variation degree between pulse wave intervals may be classified as "normal pulse".

The pulse wave analysis apparatus 301 may calculate the amplitude ratio "$AmpRatio(k) = Amp(k)_2/Amp(k)_1$" of adjacent pulse waves in the section Sk, based on the pulse waveform in the section Sk. When it is determined that the pulse waveform W does not indicate the normal pulse, the pulse wave analysis apparatus 301 performs regression analysis based on the identified combinations C1 to CK using the above equation (3) to calculate the b value (the coefficient value b) and the determination coefficient. The combination Ck (k = 1, 2, ..., K) is a combination of the amplitude ratio "$AmpRatio(k) = Amp(k)_2/Amp(k)_1$" and the pulse wave intervals $RRI(k)_1$, $RRI(k)_2$.

This may find the determination coefficient indicating the matching degree between the pulse waveform W and the model expressed as the function F representing the temporal change in the blood flow volume with heartbeat (contraction and relaxation), as well as the b value (the coefficient value b) corresponding to the refractory period of the heart. The regression analysis performed using the amplitude ratio AmpRatio(k) between adjacent pulse waves in the section Sk in place of the pulse wave amplitude may absorb the difference between the amplitudes in magnitude due to the variations in the state of the heart, measurement environment and so on at measurement.

The pulse wave analysis apparatus 301 may determine that the pulse waveform W contains noise, when the calculated determination coefficient (matching score) is less than the threshold Th. Thereby, the pulse waveform W having a low matching degree with the model representing the temporal change in the blood flow volume with heartbeat may be classified as "noise".

The pulse wave analysis apparatus 301 may determine that the pulse waveform W includes the irregular pulse, when it is determined that the pulse waveform W does not indicate the normal pulse, and the calculated determination coefficient is equal or greater than the threshold Th. Thereby, the pulse waveform W having a large variation degree between pulse wave intervals and a high matching degree with the model representing the temporal change in the blood flow volume with heartbeat may be classified as "irregular pulse".

The pulse wave analysis apparatus 301 may determine that the pulse waveform W contains noise, when the calculated b value (the coefficient value b) falls outside the predetermined range R2. Thereby, the pulse waveform W having the b value corresponding to the refractory period of the heart outside the predetermined range R2 representing the general refractory period may be classified as "irregular pulse".

The pulse wave analysis apparatus 301 may delete the combination Ck as the outlier among the combinations C1 to CK, when the determination coefficient of the calculated pulse waveform W is less than the threshold Th, or the calculated b value falls outside the predetermined range R2. This may remove the combination that greatly deviates from the general tendency of data from the combinations C1 to CK.

The pulse wave analysis apparatus 301 may delete the combination Ck as the outlier from the combinations C1 to CK using the above equation (3) and then, perform regression analysis based on the combinations after deletion, recalculating the b value and the determination coefficient. This may recalculate values of the coefficients a, b and the determination coefficient based on data with the outlier removed, improving the accuracy of pulse wave analysis.

Therefore, the pulse wave analysis apparatus 301 in accordance with Embodiment 2 may accurately classify the pulse waveform W including pulse waves in different sections S1 to SK, as "normal pulse", "irregular pulse", or "noise". Thereby, for example, even if the pulse waveform for about 10 seconds may not be ensured in one section, noise caused by body motion of the subject may be separated from the measured pulse waves to improve the analysis accuracy of pulse waves such as irregular pulse determination and pulse rate determination.

The determination method described in this embodiment may be implemented by causing a computer such as a personal computer and a workstation to perform a prepared program. The determination program is recorded in a computer-readable recording medium such as a hard disk, a flexible disk, a CD-ROM, an MO, and a DVD, and is performed by being read from the recording medium by use of the computer. The determination program may be distributed via a network such as the Internet.

REFERENCE SIGNS LIST

| | |
|---|---|
| 100 | Determination apparatus |
| 110, W, W1 to Wm, Wj | Pulse waveform |
| 300 | Pulse wave analysis system |
| 301 | Pulse wave analysis apparatus |
| 302 | Server |
| 320 | Pulse waveform DB |
| 330 | Analysis result DB |
| 340 | Individual characteristic DB |
| 801, 1801 | Acquisition unit |
| 802, 1802 | Identification unit |
| 803, 1803 | Calculation unit |
| 804, 1804 | Determination unit |
| 805, 1805 | Output unit |
| 1000, 1100 | Pulse wave analysis result |
| 1300 | Individual pulse waveform table |
| 1400 | Individual analysis result table |
| 1600 | Threshold table |

**Claims**

1. A determination apparatus (100) comprising:

   a memory; and
   a processor coupled to the memory and configured to execute control processing that includes:

   acquiring (S1701) a pulse waveform (110, W, W1 to Wm, Wj) indicating a temporal change of a measured pulse wave;
   calculating (S1709) a first value indicating a matching degree between the acquired pulse waveform (110, W, W1 to Wm, Wj) and a model expressed as a function (120), the function indicating a relationship between a beat-to-beat interval corresponding to the pulse wave interval and a blood flow volume that is proportional to the pulse wave amplitude, the function describing a model temporal course of the pulse wave amplitude during a single pulse wave interval and showing a monotonic increase during this interval, the monotonic increase starting with a section in which there is no blood flow and that corresponds to the cardiac refractory period;
   determining (S1710) whether or not the pulse waveform (110, W, W1 to Wm, Wj) contains noise based on the calculated first value; and
   outputting the obtained determination result (1400-1, 1400-2, 1400-3).

2. The determination apparatus (100) according to claim 1, wherein the control processing further includes:

   calculating (S1705) a second value indicating a variation degree between pulse wave intervals based on the pulse waveform (110, W, W1 to Wm, Wj); and
   determining (S1706) whether or not the pulse waveform (110, W, W1 to Wm, Wj) indicates a normal pulse based on the calculated second value.

3. The determination apparatus (100) according to claim 2, wherein the control processing further includes: calculating (S1709) the first value when determining that the pulse waveform (110, W, W1 to Wm, Wj) does not indicate the normal pulse.

4. The determination apparatus (100) according to claim 3, wherein the control processing further includes:

determining (S1711) that the pulse waveform (110, W, W1 to Wm, Wj) contains noise when the calculated first value is less than a threshold; and

determining (S1713) that the pulse waveform (110, W, W1 to Wm, Wj) includes an irregular pulse when the calculated first value is equal or greater than the threshold.

5. The determination apparatus (100) according to claim 1, wherein
the control processing further includes:

identifying (S1708) a plurality of pairs of pulse wave amplitude or pulse wave area and pulse wave interval from the acquired pulse waveform (110, W, W1 to Wm, Wj); and
calculating (S1709) the first value based on the identified pairs,

wherein the function defines a relationship between the pulse wave amplitude or the pulse wave area and the difference between the value of the pulse wave interval and a predetermined value corresponding to the cardiac refractory period.

6. The determination apparatus (100) according to claim 5, wherein
the control processing further includes:

performing (S1708) regression analysis based on the plurality of pairs to calculate values of the predetermined value included in the function; and
determining (S1711) that the pulse waveform (110, W, W1 to Wm, Wj) contains noise when the calculated values indicate that the predetermined value falls outside a predetermined range.

7. The determination apparatus (100) according to claim 5 or 6, wherein the control processing further includes:

deleting a pair as an outlier from the plurality of pairs when the calculated first value is less than the threshold or when the calculated predetermined value falls outside the predetermined range; and
calculating the first value based on the pairs excluding the pair as the outlier.

8. A determination apparatus (100) comprising:

a memory; and
a processor coupled to the memory and configured to execute control processing that includes:

acquiring (S2201) pulse waveforms (110, W, W1 to Wm, Wj) measured in different sections ($S_k$, $S_{k+1}$) of a pulse wave and each section indicating a temporal change of the pulse wave;
identifying (S2202, S2203) pulse wave intervals ($RRI(k)_1$, $RRI(k)_2$, $RRI(k+1)_1$, $RRI(k+1)_2$) and pulse wave amplitudes ($Amp(k)_1$, $Amp(k)_2$, $Amp(k+1)_1$, $Amp(k+1)_2$) of adjacent pulse waveforms in each of the sections ($S_k$, $S_{k+1}$);

calculating (S2204) amplitude ratios ($AmpRatio(k)=Amp(k)_2/Amp(k)_1$, $AmpRatio(k+1)=Amp(k+1)_2/Amp(k+1)_1$) of the identified pulse wave amplitudes in each of the sections ($S_k$, $S_{k+1}$);
identifying combinations ($C_k$, $C_{k+1}$) of amplitude ratio and pulse wave intervals ($C_k=(AmpRatio(k)$, $RRI(k)_1$, $RRI(k)_2)$, $C_{k+1}=(AmpRatio(k+1)$, $RRI(k+1)_1$, $RRI(k+1)_2))$ for each of the sections ($S_k$, $S_{k+1}$);
calculating (S2209) a first value based on the identified combinations, the first value indicating how much the acquired pulse waveform matches with a model expressed as a function defining a relationship between amplitude ratio and a ratio of differences between pulse wave interval and a predetermined value corresponding to the cardiac refractory period in each section ($S_k$, $S_{k+1}$);
determining (S2210) whether or not the pulse waveform (110, W, W1 to Wm, Wj) contains noise based on the calculated first value; and
outputting the obtained determination result (1400-1, 1400-2, 1400-3).

9. The determination apparatus (100) according to claim 8, wherein
the control processing further includes:

performing (S2209) regression analysis based on the combinations in the sections ($S_k$, $S_{k+1}$) to calculate the predetermined values included in the function; and

determining (S2213) that the pulse waveform (110, W, W1 to Wm, Wj) contains noise when the calculated predetermined values fall outside a predetermined range.

10. The determination apparatus (100) according to claim 8, wherein the control processing further includes:

deleting (S2211) a combination as an outlier from the combinations in the sections ($S_k$, $S_{k+1}$), when the calculated first value is less than the threshold, or when the calculated predetermined values fall outside the predetermined range; and

calculating (S2212:Yes) the first value based on the combinations excluding the combination as the outlier.

11. The determination apparatus (100) according to claim 1 or claim 8, wherein the pulse waveform (110, W, W1 to Wm, Wj) is acquired by measuring, as pulse waves of a living body, a brightness change signal included in a subject's moving image taken by use of an imaging device.

12. A determination method performed by a computer, the method comprising:

acquiring (S1701), by a processor of the computer, a pulse waveform (110, W, W1 to Wm, Wj) indicating a temporal change of the measured pulse wave;

calculating (S1709), by the processor of the computer, a first value indicating a matching degree between the acquired pulse waveform (110, W, W1 to Wm, Wj) and a model expressed as a function, the function indicating a relationship between a beat-to-beat interval corresponding to the pulse wave interval and a blood flow volume that is proportional to the pulse wave amplitude, the function describing a model temporal course of the pulse wave amplitude during a single pulse wave interval and showing a monotonic increase during this interval, the monotonic increase starting with a section in which there is no blood flow and that corresponds to the cardiac refractory period;

determining (S1710), by the processor of the computer, whether or not the pulse waveform (110, W, W1 to Wm, Wj) contains noise based on the calculated first value; and

outputting, by the processor of the computer, the obtained determination result (1400-1, 1400-2, 1400-3).

13. A computer program comprising instructions which, when the program is executed by a computer, cause the computer to carry out the steps of the method of claim 12.

14. A determination method performed by a computer, the method comprising:

acquiring (S2201) pulse waveforms (110, W, W1 to Wm, Wj) measured in different sections ($S_k$, $S_{k+1}$) of a pulse wave and each section indicating a temporal change of the pulse wave;

identifying (S2202, S2203) pulse wave intervals ($RRI(k)_1$, $RRI(k)_2$, $RRI(k+1)_1$, $RRI(k+1)_2$) and pulse wave amplitudes ($Amp(k)_1$, $Amp(k)_2$, $Amp(k+1)_1$, $Amp(k+1)_2$) of adjacent pulse waveforms in each of the sections ($S_k$, $S_{k+1}$);

calculating (S2204) amplitude ratios ($AmpRatio(k)=Amp(k)_2/Amp(k)_1$, $AmpRatio(k+1)=Amp(k+1)_2/Amp(k+1)_1$) of the identified pulse wave amplitudes in each of the sections ($S_k$, $S_{k+1}$);

identifying combinations ($C_k$, $C_{k+1}$) of amplitude ratio and pulse wave intervals ($C_k=(AmpRatio(k), RRI(k)_1$, $RRI(k)_2)$, $C_{k+1}=(AmpRatio(k+1), RRI(k+1)_1, RRI(k+1)_2))$ for each of the sections ($S_k$, $S_{k+1}$);

calculating (S2209) a first value based on the identified combinations, the first value indicating how much the acquired pulse waveform matches with a model expressed as a function defining a relationship between amplitude ratio and a ratio of differences between pulse wave interval and a predetermined value corresponding to the cardiac refractory period in each section ($S_k$, $S_{k+1}$);

determining (S2210) whether or not the pulse waveform (110, W, W1 to Wm, Wj) contains noise based on the calculated first value; and

outputting the obtained determination result (1400-1, 1400-2, 1400-3).

15. A computer program comprising instructions which, when the program is executed by a computer, cause the computer to carry out the steps of the method of claim 14.

**Patentansprüche**

1. Bestimmungseinrichtung (100), umfassend:

   einen Speicher; und
   einen Prozessor, der an den Speicher gekoppelt und zum Ausführen einer Steuerverarbeitung ausgelegt ist, die einschließt:

   Erfassen (S1701) einer Pulswellenform (110, W, W1 bis Wm, Wj), die eine zeitliche Änderung einer gemessenen Pulswelle angibt;
   Berechnen (S1709) eines ersten Werts, der einen Übereinstimmungsgrad zwischen der erfassten Pulswellenform (110, W, W1 bis Wm, Wj) und einem als eine Funktion (120) ausgedrückten Modell angibt, wobei die Funktion eine Beziehung zwischen einem Schlagzu-Schlag-Intervall entsprechend dem Pulswellenintervall und einem Blutflussvolumen, das proportional zu der Pulswellenamplitude ist, angibt, wobei die Funktion einen Modellzeitverlauf der Pulswellenamplitude während eines einzelnen Pulswellenintervalls beschreibt und einen monotonen Anstieg während dieses Intervalls anzeigt, wobei der monotone Anstieg mit einem Abschnitt beginnt, in dem kein Blutfluss vorhanden ist und der der Herzrefraktärzeit entspricht;
   Bestimmen (S1710), basierend auf dem berechneten ersten Wert, ob die Pulswellenform (110, W1 bis Wm, Wj) Rauschen enthält oder nicht; und
   Ausgeben des erhaltenen Bestimmungsergebnisses (1400-1, 1400-2, 1400-3).

2. Bestimmungseinrichtung (100) nach Anspruch 1, wobei die Steuerverarbeitung weiter einschließt:

   Berechnen (S1705) eines zweiten Werts, der einen Abweichungsgrad zwischen Pulswellenintervallen basierend auf der Pulswellenform (110, W, W1 bis Wm, Wj) angibt; und
   Bestimmen (S1706), basierend auf dem berechneten zweiten Wert, ob die Pulswellenform (110, W, W1 bis Wm, Wj) einen normalen Puls angibt oder nicht.

3. Bestimmungseinrichtung (100) nach Anspruch 2, wobei die Steuerverarbeitung weiter einschließt: Berechnen (S1709) des ersten Werts, wenn bestimmt wird, dass die Pulswellenform (110, W, W1 bis Wm, Wj) nicht den normalen Puls angibt.

4. Bestimmungseinrichtung (100) nach Anspruch 3, wobei die Steuerverarbeitung weiter einschließt:

   Bestimmen (S1711), dass die Pulswellenform (110, W, W1 bis Wm, Wj) Rauschen enthält, wenn der berechnete erste Wert kleiner als eine Schwelle ist; und
   Bestimmen (S1713), dass die Pulswellenform (110, W, W1 bis Wm, Wj) einen unregelmäßigen Puls einschließt, wenn der berechnete erste Wert größer oder gleich der Schwelle ist.

5. Bestimmungseinrichtung (100) nach Anspruch 1, wobei die Steuerverarbeitung weiter einschließt:

   Identifizieren (S1708) einer Vielzahl von Paaren von Pulswellenamplitude oder Pulswellenbereich und Pulswellenintervall aus der erfassten Pulswellenform (110, W, W1 bis Wm, Wj); und
   Berechnen (S1709) des ersten Werts basierend auf den identifizierten Paaren, wobei die Funktion eine Beziehung zwischen der Pulswellenamplitude oder dem Pulswellenbereich und der Differenz zwischen dem Wert des Pulswellenintervalls und einem vorbestimmten Wert entsprechend der Herzrefraktärzeit definiert.

6. Bestimmungseinrichtung (100) nach Anspruch 5, wobei die Steuerverarbeitung weiter einschließt:

   Durchführen (S1708) einer Regressionsanalyse basierend auf der Vielzahl von Paaren zum Berechnen von Werten des vorbestimmten Werts, der in der Funktion eingeschlossen ist; und
   Bestimmen (S1711), dass die Pulswellenform (110, W, W1 bis Wm, Wj) Rauschen enthält, wenn die berechneten Werte angeben, dass der vorbestimmte Wert außerhalb eines vorbestimmten Bereichs liegt.

7. Bestimmungseinrichtung (100) nach Anspruch 5 oder 6, wobei die Steuerverarbeitung weiter einschließt:

   Löschen eines Paars als einen Ausreißer aus der Vielzahl von Paaren, wenn der berechnete erste Wert kleiner als die Schwelle ist oder wenn der berechnete vorbestimmte Wert außerhalb des vorbestimmten Bereichs liegt;

und
Berechnen des ersten Werts basierend auf den Paaren, wobei das Paar als der Ausreißer ausgeschlossen ist.

8. Bestimmungseinrichtung (100), umfassend:

einen Speicher und
einen Prozessor, der an den Speicher gekoppelt und zum Ausführen einer Steuerverarbeitung ausgelegt ist, die einschließt:

Erfassen (S2201) von Pulswellenformen (110, W, W1 bis Wm, Wj), die in verschiedenen Abschnitten ($S_k$, $S_{k+1}$) einer Pulswelle gemessen werden, und wobei jeder Abschnitt eine zeitliche Änderung der Pulswelle angibt;
Identifizieren (S2202, S2203) von Pulswellenintervallen ($RRI(k)_1$, $RRI(k)_2$, $RRI(k+1)_1$, $RRI(k+1)_2$) und Pulswellenamplituden ($Amp(k)_1$, $Amp(k)_2$, $Amp(k+1)_1$, $Amp(k+1)_2$) von benachbarten Pulswellenformen in jedem der Abschnitte ($S_k$, $S_{k+1}$);
Berechnen (S2204) von Amplitudenverhältnissen ($AmpRatio(k)=Amp(k)_2/Amp(k)_1$, $AmpRatio(k+1)=Amp(k+1)_2/Amp(k+1)_1$) der identifizierten Pulswellenamplituden in jedem der Abschnitte ($S_k$, $S_{k+1}$);
Identifizieren von Kombinationen ($C_k$, $C_{k+1}$) von Amplitudenverhältnis und Pulswellenintervallen ($Ck=(AmpRatio(k), RRI(k)_1, RRI(k)_2)$, $C_{k+1}=(AmpRatio(k+1), RRI(k+1)_1, RRI(k+1)_2)$) für jeden der Abschnitte ($S_k$, $S_{k+1}$);
Berechnen (S2209) eines ersten Werts basierend auf den identifizierten Kombinationen, wobei der erste Wert angibt, inwieweit die erfasste Pulswellenform mit einem Modell übereinstimmt, das als eine Funktion ausgedrückt ist, die eine Beziehung zwischen einem Amplitudenverhältnis und einem Verhältnis von Differenzen zwischen einem Pulswellenintervall und einem vorbestimmten Wert entsprechend der Herzrefraktärzeit in jedem Abschnitt ($S_k$, $S_{k+1}$) definiert;
Bestimmen (S2210), basierend auf dem berechneten ersten Wert, ob die Pulswellenform (110, W, W1 bis Wm, Wj) Rauschen enthält oder nicht; und
Ausgeben des erhaltenen Bestimmungsergebnisses (1400-1, 1400-2, 1400-3).

9. Bestimmungseinrichtung (100) nach Anspruch 8, wobei die Steuerverarbeitung weiter einschließt:

Durchführen (S2209) einer Regressionsanalyse basierend auf den Kombinationen in den Abschnitten ($S_k$, $S_{k+1}$) zum Berechnen der vorbestimmten Werte, die in der Funktion eingeschlossen sind; und
Bestimmen (S2213), dass die Pulswellenform (110, W, W1 bis Wm, Wj) Rauschen enthält, wenn die berechneten vorbestimmten Werte außerhalb eines vorbestimmten Bereichs liegen.

10. Bestimmungseinrichtung (100) nach Anspruch 8, wobei die Steuerverarbeitung weiter einschließt:

Löschen (S2211) einer Kombination als einen Ausreißer aus den Kombinationen in den Abschnitten ($S_k$, $S_{k+1}$), wenn der berechnete erste Wert kleiner als die Schwelle ist oder wenn die berechneten vorbestimmten Werte außerhalb des vorbestimmten Bereichs liegen; und
Berechnen (S2212:Ja) des ersten Werts basierend auf den Kombinationen, wobei die Kombination als der Ausreißer ausgeschlossen ist.

11. Bestimmungseinrichtung (100) nach Anspruch 1 oder Anspruch 8, wobei die Pulswellenform (110, W, W1 bis Wm, Wj) durch Messen, als Pulswellen eines lebenden Körpers, eines Helligkeitsänderungssignals, das in einem Bewegtbild einer Person, das unter Verwendung einer Bildgebungsvorrichtung aufgenommen wird, eingeschlossen ist, erfasst wird.

12. Bestimmungsverfahren, das durch einen Computer durchgeführt wird, wobei das Verfahren umfasst:

Erfassen (S1701), durch einen Prozessor des Computers, einer Pulswellenform (110, W, W1 bis Wm, Wj), die eine zeitliche Änderung der gemessenen Pulswelle angibt;
Berechnen (S1709), durch den Prozessor des Computers, eines ersten Werts, der einen Übereinstimmungsgrad zwischen der erfassten Pulswellenform (110, W, W1 bis Wm, Wj) und einem als eine Funktion ausgedrückten Modell angibt, wobei die Funktion eine Beziehung zwischen einem Schlag-zu-Schlag-Intervall entsprechend dem Pulswellenintervall und einem Blutflussvolumen, das proportional zu der Pulswellenamplitude ist, angibt, wobei die Funktion einen Modellzeitverlauf der Pulswellenamplitude während eines einzelnen Pulswellenintervalls beschreibt und einen monotonen Anstieg während dieses Intervalls anzeigt, wobei der monotone Anstieg mit einem Abschnitt beginnt, in dem kein Blutfluss vorhanden ist und der der Herzrefraktärzeit entspricht; Bestimmen (S1710), durch den Prozessor des Computers, basierend auf dem berechneten ersten Wert, ob die Pulswellenform (110, W, W1 bis Wm, Wj) Rauschen enthält oder nicht; und

Ausgeben, durch den Prozessor des Computers, des erhaltenen Bestimmungsergebnisses (1400-1, 1400-2, 1400-3).

13. Computerprogramm, umfassend Anweisungen, die bei Ausführung des Programms durch einen Computer den Computer dazu veranlassen, die Schritte des Verfahrens nach Anspruch 12 auszuführen.

14. Bestimmungsverfahren, das durch einen Computer durchgeführt wird, das Verfahren umfassend:

Erfassen (S2201) von Pulswellenformen (110, W, W1 bis Wm, Wj), die in verschiedenen Abschnitten ($S_k$, $S_{k+1}$) einer Pulswelle gemessen werden, und wobei jeder Abschnitt eine zeitliche Änderung der Pulswelle angibt; Identifizieren (S2202, S2203) von Pulswellenintervallen ($RRI(k)_1$, $RRI(k)_2$, $RRI(k+1)_1$, $RRI(k+1)_2$) und Pulswellenamplituden ($Amp(k)_1$, $Amp(k)_2$, $Amp(k+1)_1$, $Amp(k+1)_2$) von benachbarten Pulswellenformen in jedem der Abschnitte ($S_k$, $S_{k+1}$); Berechnen (S2204) von Amplitudenverhältnissen ($AmpRatio(k)=Amp(k)_2/Amp(k)_1$, $AmpRatio(k+1)=Amp(k+1)_2/Amp(k+1)_1$) der identifizierten Pulswellenamplituden in jedem der Abschnitte ($S_k$, $S_{k+1}$); Identifizieren von Kombinationen ($C_k$, $C_{k+1}$) von Amplitudenverhältnis und Pulswellenintervallen ($Ck=(AmpRatio(k)$, $RRI(k)_1$, $RRI(k)_2)$, $C_{k+1}=(AmpRatio(k+1)$, $RRI(k+1)_1$, $RRI(k+1)_2)$) für jeden der Abschnitte ($S_k$, $S_{k+1}$); Berechnen (S2209) eines ersten Werts basierend auf den identifizierten Kombinationen, wobei der erste Wert angibt, inwieweit die erfasste Pulswellenform mit einem Modell übereinstimmt, das als eine Funktion ausgedrückt ist, die eine Beziehung zwischen einem Amplitudenverhältnis und einem Verhältnis von Differenzen zwischen einem Pulswellenintervall und einem vorbestimmten Wert entsprechend der Herzrefraktärzeit in jedem Abschnitt ($S_k$, $S_{k+1}$) definiert; Bestimmen (S2210), basierend auf dem berechneten ersten Wert, ob die Pulswellenform (110, W, W1 bis Wm, Wj) Rauschen enthält oder nicht; und Ausgeben des erhaltenen Bestimmungsergebnisses (1400-1, 1400-2, 1400-3).

15. Computerprogramm, umfassend Anweisungen, die bei Ausführung des Programms durch einen Computer den Computer dazu veranlassen, die Schritte des Verfahrens nach Anspruch 14 auszuführen.

## Revendications

1. Appareil de détermination (100) comprenant :

une mémoire ; et
un processeur couplé à la mémoire et configuré pour exécuter un traitement de commande qui inclut :

acquérir (S1701) une forme d'onde impulsionnelle (110, W, W1 à Wm, Wj) indiquant un changement temporel d'une onde impulsionnelle mesurée ;
calculer (S1709) une première valeur indiquant un degré de correspondance entre la forme d'onde impulsionnelle acquise (110, W, W1 à Wm, Wj) et un modèle exprimé comme une fonction (120), la fonction indiquant une relation entre un intervalle de battement à battement correspondant à l'intervalle d'onde impulsionnelle et un volume de flux sanguin qui est proportionnel à l'amplitude d'onde impulsionnelle, la fonction décrivant une course temporelle modèle de l'amplitude d'onde impulsionnelle au cours d'un seul intervalle d'onde impulsionnelle et montrant une augmentation monotone au cours de cet intervalle, l'augmentation monotone démarrant par une section dans laquelle il n'y a pas de flux sanguin et qui correspond à la période réfractaire cardiaque ;
déterminer (S1710) si la forme d'onde impulsionnelle (110, W, W1 à Wm, Wj) contient ou non du bruit sur la base de la première valeur calculée ; et
délivrer le résultat de détermination obtenu (1400-1, 1400-2, 1400-3).

2. Appareil de détermination (100) selon la revendication 1, dans lequel le traitement de commande inclut en outre :

calculer (S1705) une seconde valeur indiquant un degré de variation entre les intervalles d'ondes impulsionnelles sur la base de la forme d'onde impulsionnelle (110, W, W1 à Wm, Wj) ; et
déterminer (S1706) si la forme d'onde impulsionnelle (110, W, W1 à Wm, Wj) indique ou non une impulsion normale sur la base de la seconde valeur calculée.

3.  Appareil de détermination (100) selon la revendication 2, dans lequel le traitement de commande inclut en outre :
calculer (S1709) la première valeur lorsque l'on détermine que la forme d'onde impulsionnelle (110, W, W1 à Wm, Wj) n'indique pas l'impulsion normale.

4.  Appareil de détermination (100) selon la revendication 3, dans lequel le traitement de commande inclut en outre :

déterminer (S1711) que la forme d'onde impulsionnelle (110, W, W1 à Wm, Wj) contient du bruit lorsque la première valeur calculée est inférieure à un seuil ; et
déterminer (S1713) que la forme d'onde impulsionnelle (110, W, W1 à Wm, Wj) inclut une impulsion irrégulière lorsque la première valeur calculée est égale ou supérieure au seuil.

5.  Appareil de détermination (100) selon la revendication 1, dans lequel le traitement de commande inclut en outre :

identifier (S1708) une pluralité de paires d'amplitude d'onde impulsionnelle ou de zone d'onde impulsionnelle et d'intervalle d'onde impulsionnelle à partir de la forme d'onde impulsionnelle acquise (110, W, W1 à Wm, Wj) ; et
calculer (S1709) la première valeur sur la base des paires identifiées, dans lequel la fonction définit une relation entre l'amplitude d'onde impulsionnelle ou la zone d'onde impulsionnelle et la différence entre la valeur de l'intervalle d'onde impulsionnelle et une valeur prédéterminée correspondant à la période réfractaire cardiaque.

6.  Appareil de détermination (100) selon la revendication 5, dans lequel le traitement de commande inclut en outre :

effectuer (S1708) une analyse de régression sur la base de la pluralité de paires pour calculer des valeurs de la valeur prédéterminée incluse dans la fonction ; et
déterminer (S1711) que la forme d'onde impulsionnelle (110, W, W1 à Wm, Wj) contient du bruit lorsque les valeurs calculées indiquent que la valeur prédéterminée tombe en dehors d'une plage prédéterminée.

7.  Appareil de détermination (100) selon la revendication 5 ou 6, dans lequel le traitement de commande inclut en outre :

effacer une paire qui dépasse de la pluralité de paires lorsque la première valeur calculée est inférieure au seuil ou lorsque la valeur prédéterminée calculée tombe en dehors de la plage prédéterminée ; et
calculer la première valeur sur la base des paires à l'exclusion de la paire en tant que dépassement.

8.  Appareil de détermination (100) comprenant :

une mémoire ; et
un processeur couplé à la mémoire et configuré pour exécuter un traitement de commande qui inclut :

acquérir (S2201) des formes d'ondes impulsionnelles (110, W, W1 à Wm, Wj) mesurées dans différentes sections ($S_k$, $S_{k+1}$) d'une onde impulsionnelle et chaque section indiquant un changement temporel de l'onde impulsionnelle ;
identifier (S2202, S2203) des intervalles d'ondes impulsionnelles ($RRI(k)_1$, $RRI(k)_2$, $RRI(k+1)_1$, $RRI(k+1)_2$) et des amplitudes d'ondes impulsionnelles ($Amp(k)_1$, $Amp(k)_2$, $Amp(k+1)_1$, $Amp(k+1)_2$) de formes d'ondes impulsionnelles adjacentes dans chacune des sections ($S_k$, $S_{k+1}$) ;
calculer (S2204) des rapports d'amplitude ($AmpRatio(k) = Amp(k)_2/Amp(k)_1$, $AmpRatio(k+1) = Amp(k+1)_2/Amp(k+1)_1$) des amplitudes d'ondes impulsionnelles identifiées dans chacune des sections ($S_k$, $S_{k+1}$) ;
identifier des combinaisons ($C_k$, $C_{k+1}$) d'intervalles de rapports d'amplitude et d'ondes impulsionnelles ($C_k = (AmpRatio(k), RRI(k)_1, RRI(k)_2)$, $C_{k+1} = (AmpRatio(k+1), RRI(k+1)_1, RRI(k+1)_2)$) pour chacune des sections ($S_k$, $S_{k+1}$) ;
calculer (S2209) une première valeur sur la base des combinaisons identifiées, la première valeur indiquant de combien la forme d'onde impulsionnelle acquise correspond à un modèle exprimé comme une fonction définissant une relation entre un rapport d'amplitudes et un rapport de différences entre un intervalle d'onde impulsionnelle et une valeur prédéterminée correspondant à la période réfractaire cardiaque dans chaque section ($S_k$, $S_{k+1}$) ;

déterminer (S2210) si la forme d'onde impulsionnelle (110, W, W1 à Wm, Wj) contient ou non du bruit sur la base de la première valeur calculée ; et

délivrer le résultat de détermination obtenu (1400-1, 1400-2, 1400-3).

9. Appareil de détermination (100) selon la revendication 8, dans lequel le traitement de commande inclut en outre :

effectuer (S2209) une analyse de régression sur la base des combinaisons dans les sections ($S_k$, $S_{k+1}$) pour calculer les valeurs prédéterminées incluses dans la fonction ; et

déterminer (S2213) que la forme d'onde impulsionnelle (110, W, W1 à Wm, Wj) contient du bruit lorsque les valeurs prédéterminées calculées tombent en dehors d'une plage prédéterminée.

10. Appareil de détermination (100) selon la revendication 8, dans lequel le traitement de commande inclut en outre :

effacer (S2211) une combinaison en tant que dépassement des combinaisons dans les sections ($S_k$, $S_{k+1}$) lorsque la première valeur calculée est inférieure au seuil ou lorsque les valeurs prédéterminées calculées tombent en dehors de la plage prédéterminée ; et

calculer (S2212 :Oui) la première valeur sur la base des combinaisons à l'exclusion de la combinaison en tant que dépassement.

11. Appareil de détermination (100) selon la revendication 1 ou la revendication 8, dans lequel la forme d'onde impulsionnelle (110, W, W1 à Wm, Wj) est acquise par mesure, comme ondes impulsionnelles d'un corps vivant, d'un signal de changement de brillance inclus dans une image mobile d'un sujet prise par utilisation d'un dispositif d'imagerie.

12. Procédé de détermination effectué par un ordinateur, le procédé comprenant :

acquérir (S1701), par un processeur de l'ordinateur, une forme d'onde impulsionnelle (110, W, W1 à Wm, Wj) indiquant un changement temporel de l'onde impulsionnelle mesurée ;

calculer (S1709), par le processeur de l'ordinateur, une première valeur indiquant un degré de correspondance entre la forme d'onde impulsionnelle acquise (110, W, W1 à Wm, Wj) et un modèle exprimé comme une fonction, la fonction indiquant une relation entre un intervalle de battement à battement correspondant à l'intervalle d'onde impulsionnelle et un volume de flux sanguin qui est proportionnel à l'amplitude d'onde impulsionnelle, la fonction décrivant une course temporelle modèle de l'amplitude d'onde impulsionnelle au cours d'un seul intervalle d'onde impulsionnelle et montrant une augmentation monotone au cours de cet intervalle, l'augmentation monotone démarrant par une section dans laquelle il n'y a pas de flux sanguin et qui correspond à la période réfractaire cardiaque ;

déterminer (S1710), par le processeur de l'ordinateur, si la forme d'onde impulsionnelle (110, W, W1 à Wm, Wj) contient ou non du bruit sur la base de la première valeur calculée ; et

délivrer, par le processeur de l'ordinateur, le résultat de détermination obtenu (1400-1, 1400-2, 1400-3).

13. Programme d'ordinateur comprenant des instructions qui, lorsque le programme est exécuté par un ordinateur, amènent l'ordinateur à effectuer les étapes du procédé de la revendication 12.

14. Procédé de détermination effectué par un ordinateur, le procédé comprenant :

acquérir (S2201) des formes d'ondes impulsionnelles (110, W, W1 à Wm, Wj) mesurées dans différentes sections ($S_k$, $S_{k+1}$) d'une onde impulsionnelle et chaque section indiquant un changement temporel de l'onde impulsionnelle ;

identifier (S2202, S2203) des intervalles d'ondes impulsionnelles ($RRI(k)_1$, $RRI(k)_2$, $RRI(k+1)_1$, $RRI(k+1)_2$) et des amplitudes d'ondes impulsionnelles ($Amp(k)_1$, $Amp(k)_2$, $Amp(k+1)_1$, $Amp(k+1)_2$) de formes d'ondes impulsionnelles adjacentes dans chacune des sections ($S_k$, $S_{k+1}$) ;

calculer (S2204) des rapports d'amplitude ($AmpRatio(k) = Amp(k)_2/Amp(k)_1$, $AmpRatio(k+1) = Amp(k+1)_2/Amp(k+1)_1$) des amplitudes d'ondes impulsionnelles identifiées dans chacune des sections ($S_k$, $S_{k+1}$) ;

identifier des combinaisons ($C_k$, $C_{k+1}$) d'intervalles de rapports d'amplitude et d'ondes impulsionnelles ($C_k = (AmpRatio(k), RRI(k)_1, RRI(k)_2)$, $C_{k+1} = (AmpRatio(k+1), RRI(k+1)_1, RRI(k+1)_2)$) pour chacune des sections ($S_k$, $S_{k+1}$) ;

calculer (S2209) une première valeur sur la base des combinaisons identifiées, la première valeur indiquant de combien la forme d'onde impulsionnelle acquise correspond à un modèle exprimé comme une fonction définissant une relation entre un rapport d'amplitude et un rapport de différences entre un intervalle d'onde impulsionnelle et une valeur prédéterminée correspondant à la période réfractaire cardiaque dans chaque section ($S_k$, $S_{k+1}$) ;

déterminer (S2210) si la forme d'onde impulsionnelle (110, W, W1 à Wm, Wj) contient ou non du bruit sur la base de la première valeur calculée ; et

délivrer le résultat de détermination obtenu (1400-1, 1400-2, 1400-3).

**15.** Programme d'ordinateur comprenant des instructions qui, lorsque le programme est exécuté par un ordinateur, amènent l'ordinateur à effectuer les étapes du procédé de la revendication 14.

# FIG. 1

# FIG. 2

PULSE —— PULSE WAVE  ⟷ INTERVAL  ⟵----⟶ AMPLITUDE

PULSE WAVE SIGNAL

MEASUREMENT TIME

110

INTERVAL

PEAK DETECTION

AMPLITUDE

MAXIMUM VALUE

PEAK DETECTION

MINIMUM VALUE

EP 3 372 152 B1

# FIG. 3

# FIG. 4

# FIG. 5

# FIG. 6

PULSE WAVEFORM DB ~320

| WAVEFORM ID | PEAK DETECTION TIME | AMPLITUDE | PULSE WAVE INTERVAL | |
|---|---|---|---|---|
| W1 | $t(1)_1$ | $Amp(1)_1$ | $RRI(1)_1$ | ~600-1 |
| | $t(1)_2$ | $Amp(1)_2$ | $RRI(1)_2$ | ~600-2 |
| | ... | ... | ... | |
| W2 | $t(2)_1$ | $Amp(2)_1$ | $RRI(2)_1$ | |
| | ... | ... | ... | |
| : | : | : | : | |
| Wj | $t_1$ | $Amp_1$ | $RRI_1$ | |
| | $t_2$ | $Amp_2$ | $RRI_2$ | |
| | : | : | : | |
| | $t_i$ | $Amp_i$ | $RRI_i$ | |
| | : | : | : | |
| | $t_n$ | $Amp_n$ | $RRI_n$ | |
| : | : | : | : | |
| Wm | ... | ... | ... | |

AMPLITUDE

$Amp_{i+1}$

$Amp_i$

$RRI_i$  $RRI_{i+1}$

TIME

# FIG. 7

ANALYSIS
RESULT DB /330

| ANALYSIS TIME | DETERMINATION COEFFICIENT | b VALUE | DETERMINATION RESULT |
|---|---|---|---|
| T1 | – | – | NORMAL PULSE |
| T2 | 0.56 | 288 | IRREGULAR PULSE |
| T3 | 0.33 | 255 | NOISE |
| : | : | : | : |

700-1

700-2

700-3

# FIG. 8

EP 3 372 152 B1

# FIG. 9

# FIG. 10

PULSE WAVE
ANALYSIS
RESULT — 1000

| ANALYSIS TIME | DETERMINATION RESULT |
|---|---|
| T1 | NORMAL PULSE |
| T2 | IRREGULAR PULSE |
| T3 | NOISE |
| : | : |
| Tm | ... |

700-1
700-2
700-3

# FIG. 11

PULSE WAVE
ANALYSIS
RESULT                    1100

| PEAK DETECTION TIME | DETERMINATION RESULT |
|---------------------|----------------------|
| 13:01:00 | NORMAL PULSE |
| 13:01:01 | IRREGULAR PULSE |
| 13:01:03 | IRREGULAR PULSE |
| : | : |
| 14:13:49 | NOISE |

# FIG. 12

# FIG. 13

INDIVIDUAL PULSE WAVEFORM TABLE — 1300

| USER ID | PEAK DETECTION TIME | AMPLITUDE | PULSE WAVE INTERVAL | |
|---------|--------------------|-----------|--------------------|---|
| | 13:01:00 | 1.1 | 890 | ~1300-1 |
| | 13:01:01 | 1.6 | 1651 | ~1300-2 |
| U1 | 13:01:03 | 1.2 | 780 | ~1300-3 |
| | : | : | : | |
| | 14:13:49 | 0.9 | 900 | |

# FIG. 14

<table>
<tr><td colspan="5" align="center">INDIVIDUAL ANALYSIS<br>RESULT TABLE 〜1400</td></tr>
<tr><td>USER ID</td><td>ANALYSIS<br>TIME</td><td>DETERMINATION<br>COEFFICIENT</td><td>b VALUE</td><td>DETERMINATION<br>RESULT</td></tr>
<tr><td rowspan="5">U1</td><td>13:01:10</td><td>-</td><td>-</td><td>NORMAL<br>PULSE</td></tr>
<tr><td>13:01:13</td><td>0.42</td><td>288</td><td>NOISE</td></tr>
<tr><td>13:01:14</td><td>0.33</td><td>255</td><td>NOISE</td></tr>
<tr><td>:</td><td>:</td><td>:</td><td>:</td></tr>
<tr><td>14:13:30</td><td>0.3</td><td>20</td><td>NOISE</td></tr>
</table>

1400-1
1400-2
1400-3

EP 3 372 152 B1

FIG. 15

# FIG. 16

THRESHOLD TABLE   1600

| USER ID | Th | R2 |
|---------|-----|--------|
| U1 | 0.3 | 50-400 |

1600-1

# FIG. 17A

```
                    ┌─────────────┐
                    │    START    │
                    └──────┬──────┘
                           │
S1701 ┌────────────────────▼────────────────────┐
      │         ACQUIRE PULSE WAVEFORM           │
      └────────────────────┬────────────────────┘
                           │
S1702 ┌────────────────────▼────────────────────┐
      │       IDENTIFY PULSE WAVE INTERVAL       │
      └────────────────────┬────────────────────┘
                           │
S1703 ┌────────────────────▼────────────────────┐
      │            IDENTIFY AMPLITUDE            │
      └────────────────────┬────────────────────┘
                           │
S1704 ┌────────────────────▼────────────────────┐
      │    CALCULATE PULSE WAVE INTERVAL RATIO   │
      └────────────────────┬────────────────────┘
                           │
                           ▼
S1705            ╱ PULSE WAVE INTERVAL ╲          NO
            ╱ RATIO IS WITHIN PREDETERMINED ╲────────┐
            ╲        RANGE R1?             ╱         │
                   ╲              ╱                  ▼
                        │ YES                      ⬡ A
S1706 ┌────────────────▼────────────────────────┐
      │         DETERMINE AS NORMAL PULSE        │
      └────────────────────┬────────────────────┘
                           │
S1707 ┌────────────────────▼────────────────────┐
      │        RECORD DETERMINATION RESULT       │
      └────────────────────┬────────────────────┘
                           │
                    ┌──────▼──────┐
                    │     END     │
                    └─────────────┘
```

# FIG. 17B

A

S1708

IDENTIFY MULTIPLE PAIRS OF AMPLITUDE
AND PULSE WAVE INTERVAL

S1709

CALCULATE b VALUE AND
DETERMINATION COEFFICIENT BY
REGRESSION ANALYSIS

S1710

DETERMINATION
COEFFICIENT IS LESS THAN
THRESHOLD?

YES

NO

S1712

b VALUE FALLS
WITHIN PREDETERMINED
RANGE R2?

NO

YES

S1713

DETERMINE AS IRREGULAR PULSE

S1711

DETERMINE AS NOISE

S1714

RECORD DETERMINATION RESULT

END

FIG. 18

ACQUISITION UNIT 1801 — IDENTIFICATION UNIT 1802 — CALCULATION UNIT 1803 — DETERMINATION UNIT 1804 — OUTPUT UNIT 1805

301

# FIG. 19

# FIG. 20

# FIG. 21

DETERMINATION COEFFICIENT
= 0.89

# FIG. 22A

```
                    ┌─────────────┐
                    │    START    │
                    └──────┬──────┘
                           │
 S2201 ┌───────────────────▼────────────────────┐
       │        ACQUIRE PULSE WAVEFORM           │
       └───────────────────┬────────────────────┘
                           │
 S2202 ┌───────────────────▼────────────────────┐
       │      IDENTIFY PULSE WAVE INTERVAL       │
       └───────────────────┬────────────────────┘
                           │
 S2203 ┌───────────────────▼────────────────────┐
       │           IDENTIFY AMPLITUDE            │
       └───────────────────┬────────────────────┘
                           │
 S2204 ┌───────────────────▼────────────────────┐
       │        CALCULATE AMPLITUDE RATIO        │
       └───────────────────┬────────────────────┘
                           │
 S2205 ┌───────────────────▼────────────────────┐               ╱C╲
       │   CALCULATE PULSE WAVE INTERVAL RATIO   │
       └───────────────────┬────────────────────┘
                           │◄───────────────────────────────────┘
                           │
 S2206              ╱──────▼──────╲
               ╱  PULSE WAVE INTERVAL  ╲        NO
            ◄  RATIO IS WITHIN PREDETERMINED ─────────────┐
               ╲     RANGE R1?        ╱                    │
                 ╲─────┬───────╲                           │
                   YES │                                 ╱B╲
 S2207 ┌───────────────▼────────────────────────┐
       │        DETERMINE AS NORMAL PULSE        │
       └───────────────────┬────────────────────┘
                           │
 S2208 ┌───────────────────▼────────────────────┐
       │       RECORD DETERMINATION RESULT       │
       └───────────────────┬────────────────────┘
                           │
                    ┌──────▼──────┐
                    │     END     │
                    └─────────────┘
```

# FIG. 22B

```
                          ┌─B─┐
                          └─┬─┘
                            ↓
S2209 ┌──────────────────────────────────┐
      │      CALCULATE b VALUE AND        │
      │   DETERMINATION COEFFICIENT BY    │
      │       REGRESSION ANALYSIS         │
      └──────────────────────────────────┘
                            ↓
S2210            DETERMINATION              YES
            COEFFICIENT IS LESS THAN  ─────────────────┐
                  THRESHOLD?                            │
                            │                           │
                           NO                           │
                            ↓                           │
S2214             b VALUE IS                NO          │
              WITHIN PREDETERMINED  ──────────────┐     │
                    RANGE R2?                      │     │
                            │                      │     │
                          YES                      ↓     │
                                                         ↓
                                    S2211 ┌──────────────────────────┐
                                          │      DELETE OUTLIER       │
                                          └──────────────────────────┘
                                                         ↓
                                    S2212      NUMBER OF          YES
                                          COMBINATIONS IS THREE ──────┐
                                              OR MORE?                 │
                                                   │                 ┌─┴─┐
                                                  NO                 │ C │
                                                                     └───┘
S2215 ┌──────────────────────────────┐   S2213 ┌──────────────────────────┐
      │ DETERMINE AS IRREGULAR PULSE  │         │     DETERMINE AS NOISE    │
      └──────────────────────────────┘         └──────────────────────────┘
                     │                                       │
S2216 ┌──────────────────────────────┐                      │
      │   RECORD DETERMINATION RESULT │←─────────────────────┘
      └──────────────────────────────┘
                     ↓
                 (  END  )
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009183715 A
- JP 2012161556 A
- WO 9738626 A
- JP 2010075461 A
- JP 2007125366 A
- JP 2014200390 A